# EUROPEAN PATENT APPLICATION

(11) **EP 4 321 608 A1**
(43) Date of publication of application: **14.02.2024**
(21) Application number: 22784602.9
(22) Date of filing: 30.03.2022
(51) Int. Cl.: C12N 1/15, C12N 9/42, C12N 15/31, C12P 21/00

(54) **MODIFIED FILAMENTOUS FUNGUS, AND METHOD FOR PRODUCING PROTEIN USING SAME**

(30) Priority: 09.04.2021 JP 2021066415
(71) Applicant: Kao Corporation, Chuo-ku Tokyo 103-8210 (JP)
(72) Inventor: ARAI, Toshiharu, Wakayama-shi, Wakayama 640-8580 (JP); KODAMA, Hiroshi, Haga-gun, Tochigi 321-3497 (JP)
(74) Representative: Vossius & Partner Patentanwälte Rechtsanwälte mbB
(86) International application number: PCT/JP2022/015855
(87) International publication number: WO 2022/215618

(57) **Abstract**

Provided are a modified filamentous fungus with improved protein productivity and a method for producing a protein using the modified filamentous fungus. The modified filamentous fungus expresses an ACE3 variant that is a variant in which substantially the whole of a Zn(II)₂Cys₆-type DNA-binding domain of the ACE3 is deleted. The method for producing a protein includes culturing the modified filamentous fungus.

## Description

### Field of the Invention

The present invention relates to a modified filamentous fungus and a method for producing a protein using the fungus.

### Background of the Invention

Filamentous fungus is a plant polysaccharide-degrading fungus which produce various types of cellulases and hemicellulases. In particular, *Trichoderma* can produce cellulase and hemicellulase simultaneously in large quantities and is therefore attracting attention as a microorganism for producing cellulase-based biomass-degrading enzymes.

It is desirable that the carbon source for industrial culture of microorganisms is inexpensive and soluble. Conventionally, glucose has been used widely as a carbon source in the culture of microorganisms. In contrast, the production of a protein, such as an enzyme, by a microorganism may require an inducer. For example, in cellulase production by a microorganism, an inducer is generally essential. In *Trichoderma,* expression of main cellulase genes cbh1, cbh2, egl1, and egl2 is induced by an inducer such as cellulose or cellobiose (Non Patent Literature 1). When no inducer is used, for example, when only glucose is used as a single carbon source, in general, a saccharification enzyme is hardly produced in *Trichoderma.*

As a microbiological protein production method using an inducer, a cellulase production method using Avicel, which is microcrystalline cellulose, is known. A cellulase-producing method using soluble lactose without using cellulose (Patent Literature 1) and a method for inducing cellulase production by synthesizing an inducible such as sophorose and gentiobiose from glucose by reacting cellulase (including β-glucosidase, endoglucanase, and cellobiohydrolase) derived from *Trichoderma* and glucose at high temperature (Patent Literature 2) have been disclosed. However, the cellulose substrates are expensive, and many of them are insoluble and therefore apply a load to the industrial process. Consequently, the use for industrial purposes is difficult in terms of cost and facilities. In addition, cellulase production using another inducible sugars also has disadvantages in terms of cost and process load.

Accordingly, analysis of the cellulase expression mechanism is in progress toward the production of a microorganism that can express cellulase and xylanase without using an inducer by modification of the transcriptional regulator. Previously reported positive transcription factors involved in cellulase inducible expression of *Trichoderma* include XYR1, ACE2, ACE3, and HAP2/3/5 (Non Patent Literature 2). ACE3 is a transcription factor that controls a promotor such as cbh1 which is a main cellulase. ACE3 binds to a promoter through a Zn(II)₂Cys₆-type DNA-binding domain (DBD) on the 120th to 160th amino acids on the N-terminal side (Non Patent Literature 3). Non Patent Literature 3 suggests that the interaction between ACE3 and XYR1 regulates the cellulase gene expression of *Trichoderma reesei.*

Patent Literature 3 and Non Patent Literature 4 disclose a method for increasing or decreasing the productivity of cellulase or the like in *Trichoderma reesei* by increasing or decreasing the expression of the tre77513 (ACE3) gene. Patent Literature 4 and Non Patent Literature 5 report that a filamentous fungus showing improved expression of modified ACE3, in which all six cysteines of the Zn(II)₂Cys₆-type DNA-binding domain on the N-side are retained and 7 to 17 amino acids on the C-terminal are deleted, showed improved expression of cellulase even in the absence of an inducer. Non Patent Literature 5 also describes a filamentous fungus that shows improved expression of the C-terminal-deleted ACE3 and co-expresses the wild-type or A824V mutant of XYR1. However, the effect of the XYR1 co-expression on the cellulase expression in this filamentous fungus is slightly observed in the presence of an inducer, but is not observed in the absence of an inducer.

Non Patent Literature 6 reports that deregulation of xylanase occurs in *Trichoderma* having A824V mutation of XYR1 to increase the cellulase production. Non Patent Literature 7 reports that in a *Trichoderma* strain, protein productivity in a medium containing glucose or sucrose as a carbon source is improved by combining V821F mutation in XYR1 and improved expression of ACE2.

(Patent Literature 1) Japanese Patent No. 6169077
(Patent Literature 2) Japanese Patent No. 5366286
(Patent Literature 3) U.S. Patent No. 9512415
(Patent Literature 4) WO 2018/067599

(Non Patent Literature 1) Curr. Genomics, 2013, 14:230-249
(Non Patent Literature 2) BMC Genomics, 2015, 16:326
(Non Patent Literature 3) J Biol Chem., 2019, doi:10.1074/jbc.RA119.008497
(Non Patent Literature 4) Biotech. Biofuels, 2014, 7:14
(Non Patent Literature 5) Biotech. Biofuels, 2020, 13:137
(Non Patent Literature 6) Biotech. Biofuels, 2013, 6:62
(Non Patent Literature 7) Biotech. Biofuels, 2017, 10:30

### Summary of the Invention

The present invention provides a modified filamentous fungus, which expresses an ACE3 variant, wherein
the ACE3 variant is a variant in which substantially the whole of a Zn(II)₂Cys₆-type DNA-binding domain of ACE3 is deleted.

In addition, the present invention provides a method for producing a protein, including culturing the modified filamentous fungus.

The present invention also provides a method for producing a modified filamentous fungus, including:
modifying a parent filamentous fungus so as to express an ACE3 variant, wherein
the ACE3 variant is a variant in which substantially the whole of a Zn(II)₂Cys₆-type DNA-binding domain of ACE3 is deleted.

### Brief Description of Drawings

[Figure 1] Figure 1 shows structures of ACE3 variants expressed by modified filamentous fungi.
[Figure 2] Figure 2 shows structures of ACE3 variants expressed by modified filamentous fungi.
[Figure 3] Figure 3 shows effects of DBD deletion of ACE3 on protein productivity: (A) the value on the vertical axis represents relative protein productivity of a modified filamentous fungus that expresses an ACE3 variant, and 1, 2, and 3 on the horizontal axis represent three modified filamentous fungus strains expressing the same ACE3 variant; (B) gel electrophoresis images of cultures of modified filamentous fungi.
[Figure 4] Figure 4 shows alignments of amino acid sequences of ACE3 variants derived from various *Trichoderma* fungi.
[Figure 5] Figure 5 shows protein production in modified filamentous fungi expressing ACE3 variants derived from various *Trichoderma* fungi: (A) relative protein productivity; (B) gel electrophoresis images of cultures of modified filamentous fungi.
[Figure 6] Figure 6 shows the effects of C-terminal deletion of ACE3 on protein productivity: (A) relative protein productivity of modified filamentous fungi expressing ACE3 variants; (B) gel electrophoresis images of cultures of modified filamentous fungi.
[Figure 7] Figure 7 shows the effects of co-expression of mutated XYR1 and an ACE3 variant on protein productivity: (A) gel electrophoresis images of cultures of modified filamentous fungi; (B) the protein composition ratios in cultures of modified filamentous fungi.
[Figure 8] Figure 8 shows the effects of mutated XYR1 expression and combination of DBD deletion and C-terminal deletion of ACE3 on protein productivity: (A) gel electrophoresis images of cultures of modified filamentous fungi; (B) the protein composition ratios of cultures of modified filamentous fungi.

### Detailed Description of the Invention

All Patent Literatures, Non Patent Literatures, and other publications cited herein are hereby incorporated by reference in their entirety.

As used herein, the identity in an amino acid sequence or a nucleotide sequence is calculated by a Lipman-Pearson method (Science, 1985, 227:1435-1441). Specifically, the identity is calculated by performing analysis using the homology analysis (Search homology) program of genetic information processing software Genetyx-Win (Ver. 5.1.1, Software Development Co., Ltd.) and setting the Unit size to compare (ktup) to 2.

As used herein, the term "at least 90% identity" in the context of an amino acid sequence and a nucleotide sequence refers to an identity of 90% or more, preferably 92% or more, more preferably 94% or more, further preferably 95% or more, further preferably 96% or more, further preferably 98% or more, and still preferably 99% or more.

As used herein, the term "one to several" that is used in the context of deletion, substitution, addition, or insertion of amino acid or nucleotide in an amino acid sequence or a nucleotide sequence can mean, for example, from 1 to 20, preferably from 1 to 16, more preferably from 1 to 12, further preferably from 1 to 8, and further preferably from 1 to 4, unless otherwise defined. As used herein, the "addition" of amino acid or nucleotide includes addition of one to several amino acids or nucleotides to one end and both ends of a sequence. As used herein, the "insertion" of amino acid or nucleotide includes insertion of amino acid or nucleotide into the 5' side or the 3' side of a predetermined position.

As used herein, the "corresponding position" or "corresponding region" in an amino acid sequence or a nucleotide sequence can be determined by aligning (alignment of) a target sequence and a reference sequence (for example, the amino acid sequence of SEQ ID NO: 1) so as to give a maximum homology. The alignment of an amino acid sequence or nucleotide sequence can be performed using a known algorithm, and the procedure thereof is known to those skilled in the art. For example, the alignment can be performed using the Clustal W Multiple Alignment program (Thompson, J. D. et al, 1994, Nucleic Acids Res. 22:4673-4680) at the default setting. The Clustal W can be used on the website of, for example, the European Bioinformatics Institute:EBI [www.ebi.ac.uk/index.html] or the DNA Data Bank of Japan (DDBJ [www.ddbj.nig.ac.jp/searches-j.html]) managed by the National Institute of Genetics. The position of a target sequence aligned to an arbitrary position of a reference sequence by the above-mentioned alignment is regarded as the "position corresponding" to the arbitrary position. A region between corresponding positions or a region consisting of a corresponding motif is regarded as a "corresponding region".

Those skilled in the art can further finely adjust the alignment of an amino acid sequence obtained above to optimize it. Such optimized alignment is preferably determined by considering, for example, the similarity of amino acid sequences and the frequency of insertion of a gap. Here, the similarity of amino acid sequences refers to the proportion (%) of the number of positions at which the same or analogous amino acid is present in both two aligned amino acid sequences relative to the number of full-length amino acids. The analogous amino acids are amino acids having similar properties to each other in polarity and charge, more specifically, capable of causing conservative substitution, among the 20 amino acids constituting proteins. The groups consisting of such analogous amino acids are well known to those skilled in the art, and examples thereof include, but not limited to, arginine and lysine; glutamic acid and aspartic acid; serine and threonine; glutamine and asparagine; and leucine and isoleucine.

As used herein, the term "amino acid" means 20 amino acids constituting proteins, i.e., alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V).

As used herein, the term "operable linkage" between a control region, such as a promoter, and a gene means that the gene and the control region are linked to each other such that the gene can be expressed under the control of the control region. The procedure of the "operable linkage" between a gene and a control region is known to those skilled in the art.

As used herein, "upstream" and "downstream" in the context of a gene refer to upstream and downstream in the transcription direction of the gene. For example, "a gene located downstream of a promoter" means that the gene is present on the 3' side of the promoter in the DNA sense chain, and upstream of a gene means the region on the 5' side of the gene in the DNA sense chain.

As used herein, the terms "intrinsic" and "native" used for function, property, and trait of a cell are used for representing that the function, property, and trait are originally present in the cell. In contrast, the term "exogenous" is used for representing that the function, property, and trait are not originally present in the cell but introduced from the outside. For example, an "exogenous" gene or polynucleotide is a gene or polynucleotide introduced into a cell from the outside. The exogenous gene or polynucleotide may be derived from an organism of the same species as the cell into which the gene or polynucleotide has been introduced, or may be derived from an organism of a different species (that is, a heterologous gene or polynucleotide).

The present invention provides a modified filamentous fungus, a method for producing the fungus, and a method for producing a protein using the modified filamentous fungus.

The present inventors found that a modified filamentous fungus expressing an ACE3 variant, in which a Zn(II)₂Cys₆-type DNA-binding domain (hereinafter, also simply referred to as "DBD") is deleted, shows enhanced production of a protein such as cellulase and hemicellulase without using an inducer which has been conventionally essential for production of cellulase or hemicellulase.

The modified filamentous fungus provided by the present invention can efficiently produce a protein even in an environment in which the main carbon source is a cellulase noninducible carbon source such as glucose. The filamentous fungus can efficiently produce a protein, such as cellulase and hemicellulase, even not using an expensive cellulase inducer. According to the present invention, it is possible to increase the efficiency and decrease the cost of protein production using a filamentous fungus.

The present invention relates to improvement in the protein productivity of a filamentous fungus. Conventionally, when a filamentous fungus is cultured in the presence of glucose, the protein productivity is decreased by catabolite repression in some cases. In particular, expression of a cellulase-based biomass-degrading enzyme, such as cellulase and hemicellulase, in a filamentous fungus is, in general, necessarily induced by a cellulase inducer such as cellulose, sophorose, and cellooligosaccharide (cellobiose, cellotriose, cellotetraose, cellopentaose, cellohexaose, and the like.). In contrast, the expression induction is suppressed in the presence of glucose.

The induced expression of the cellulase-based biomass-degrading enzyme by the inducer in a filamentous fungus is controlled by a large number of transcription factors such as XYR1, Cre1, ACE1, ACE2, ACE3, and HAP2/3/5. ACE3 is a transcriptional activator of cellulase and hemicellulase in a filamentous fungus. ACE3 is indispensable for transcription of the cellulase gene during lactose induction and of a part of xylanase genes. In addition, ACE3 is also partially involved in transcriptional regulation of the xyr1 gene. ACE3 of *Trichoderma reesei* has been registered in the ncbi database (www.ncbi.nlm.nih.gov/]) as NCBI Reference Sequence: QEM24913.1. Here, ACE3 consists of the amino acid sequence of SEQ ID NO: 1 and is prescribed as a polypeptide encoded by the nucleotide sequence of SEQ ID NO: 6. According to NCBI Reference Sequence:
XP_006966092.1 [www.ncbi.nlm.nih.gov/protein/XP_006966092.1], the region from positions 523 to 734 in the amino acid sequence of SEQ ID NO: 1 is assumed to interact with XYR1, the region from positions 391 to 522 is assumed to be a filamentous fungus-specific transcription factor domain, and the region from positions 120 to 160 is assumed to be a Zn(II)₂Cys₆-type DNA-binding domain (DBD). The gene coding for the DBD is divided into two regions by an intron. One (Exon2) of them codes for a region (C2) including two cysteines on the N-side of the DBD, and the other (Exon3) codes for a region (C4) including residual four cysteines. ACE3 having an equivalent structure exists also in other *Trichoderma* fungi.

It has been reported that deletion of the C-terminal amino acid of ACE3 allows the filamentous fungus to produce a protein without using an inducer (Non Patent Literature 5). However, Non Patent Literature 5 discloses that this effect by the C-terminal deletion disappears by deletion of C2 of the DBD.

In contrast, the present inventors found that an ACE3 variant in which not only C2 but also C4 of the DBD have been deleted provides an ability of expressing cellulase and hemicellulase without using an inducer to a filamentous fungus, and the filamentous fungus can highly express a protein, such as cellulase and hemicellulase, in the presence of a cellulase noninducible carbon source, such as glucose. Furthermore, this deletion of C2 and C4 in ACE3 combined with C-terminal deletion synergistically improves the protein productivity.

Accordingly, in an aspect, the present invention provides a modified filamentous fungus that expresses an ACE3 variant in which substantially the whole of the DBD is deleted and a method for producing the modified filamentous fungus. The modified filamentous fungus of the present invention can be produced by modifying a parent filamentous fungus so as to express the ACE3 variant in which substantially the whole of the DBD is deleted. The produced modified filamentous fungus of the present invention expresses the ACE3 variant in which substantially the whole of the DBD is deleted.

The DBD of ACE3 is located on the N-terminal side of the amino acid sequence of ACE3, specifically, in a region corresponding to amino acids at positions 120 to 160 in SEQ ID NO: 1, in which positions 120 to 131 are the C2, and positions 132 to 160 are the C4. As used herein, the term "deletion of substantially the whole of the DBD" refers to that the DBD has deletion of a part or the whole of C2 and deletion of a part or the whole of C4 and preferably that the DBD has deletion of the whole of C2 and a part or the whole of C4. Here, "a part of C4" preferably refers to a region including at least two cysteines in C4, more preferably a region including at least three cysteines in C4, and further preferably a region including four cysteines in C4, for example, the region corresponding to amino acids at positions 132 to 151 in SEQ ID NO: 1. Herein, examples of the "deletion of substantially the whole of the DBD" include a state in which 80% or more, preferably 90% or more, and more preferably 95% or more of the amino acid sequence of the DBD are deleted and six cysteines in the DBD are deleted.

The parent filamentous fungus of the modified filamentous fungus of the present invention is a filamentous fungus to be prepared into the modified filamentous fungus of the present invention by modification for expressing an ACE3 variant having deletion of substantially the whole of the DBD. The parent filamentous fungus is preferably a fungus that inherently expresses ACE3. For example, the parent filamentous fungus preferably expresses native ACE3 or its variant that includes the whole of the DBD. More preferably, the parent filamentous fungus is a fungus that expresses native ACE3 or its variant including the whole of the DBD and has a cellulase activity.

Examples of the parent filamentous fungus used in the present invention include, but not limited to, filamentous fungi belonging to the division Eumycota or the division Oomycota. More specifically, the examples include filamentous fungi of *Trichoderma, Aspergillus, Penicillium, Neurospora, Fusarium, Chrysosporium, Humicola, Emericella, Hypocrea, Acremonium, Myceliophthora, Piromyces, Talaromyces, Thermoascus,* and *Thielavia.* Among these filamentous fungi, filamentous fungi of *Trichoderma* are preferable.

Examples of the filamentous fungus of *Trichoderma* (hereinafter, also referred to as *Trichoderma* fungus) include *Trichoderma reesei, Trichoderma longibrachiatum, Trichoderma harzianum, Trichoderma koningii, Trichoderma viride,* and *Trichoderma atroviride,* and preferable examples are *Trichoderma reesei* and its mutant strain. For example, *Trichoderma reesei* QM9414 strain and its mutant strain, preferably *Trichoderma reesei* PC-3-7 strain (ATCC66589), *Trichoderma reesei* PCD-10 strain (FERM P-8172), *Trichoderma reesei* E1AB1 strain (hereinafter, also referred to as JN13 strain), or a mutant strain thereof, can be preferably used as a parent filamentous fungus. The E1AB1 strain is a strain in which β-glucosidase (BGL) derived from *Aspergillus aculeatus* is expressed using an egl1 promoter against a *Trichoderma reesei* PC-3-7 strain (see Enzyme and Microbial Technology, (2016), 82: 89-95 and Examples 1 to 3 of WO2013/115305).

The ACE3 variant expressed by the modified filamentous fungus of the present invention can be obtained by modifying a parent ACE3 so that substantially the whole of the DBD is deleted. The parent ACE3 can be native ACE3 or its variant that includes a part or the whole of the DBD. Preferably, the parent ACE3 can be native ACE3 or its variant that includes at least C4 of the DBD. Preferably, the parent ACE3 is ACE3 derived from *Trichoderma* fungus or its variant. Examples of the *Trichoderma* fungus can be those described above and preferably include *Trichoderma reesei, Trichoderma harzianum,* and *Trichoderma atroviride.*

Preferable examples of the parent ACE3 include ACE3 and its variants each consisting of any of the amino acid sequences of SEQ ID NOs: 1 to 4. SEQ ID NO: 1 represents the amino acid sequence of the full-length ACE3 of *Trichoderma reesei.* SEQ ID NO: 2 represents an amino acid sequence of *Trichoderma reesei* ACE3 variant with a partial DBD (C2) deletion. SEQ ID NO: 3 represents an amino acid sequence of *Trichoderma atroviride* ACE variant with a partial DBD (C2) deletion. SEQ ID NO: 4 represents an amino acid sequence of *Trichoderma harzianum* ACE3 variant with a partial DBD (C2) deletion.

Other preferable examples of the parent ACE3 include polypeptides each consisting of an amino acid sequence having at least 90% identity to any of the amino acid sequences of SEQ ID NOs: 1 to 4. Other preferable examples of the parent ACE3 include polypeptides each consisting of an amino acid sequence obtained by deletion, substitution, addition or insertion of one to several amino acids in any of the amino acid sequences of SEQ ID NOs: 1 to 4. These polypeptides of the parent ACE3 include sequences corresponding to a part or the whole of the DBD of native ACE3 (for example, SEQ ID NO: 1) and preferably can function as transcriptional activators of cellulase and hemicellulase as in native ACE3.

In the ACE3 variant expressed by the modified filamentous fungus of the present invention, substantially the whole of the DBD is deleted. Preferably, the ACE3 variant has deletion of at least a region corresponding to amino acids at positions 120 to 160 in SEQ ID NO: 1. Furthermore, the ACE3 variant may have deletion of a region on the N-terminal side than the DBD or a region on the C-terminal side than the DBD. In one embodiment, the ACE3 variant has deletion of a region corresponding to amino acids at positions 120 to 151 in SEQ ID NO: 1. In one embodiment, the ACE3 variant has deletion of a region corresponding to amino acids at positions 1 to 151 in SEQ ID NO: 1. In one embodiment, the ACE3 variant has deletion of a region corresponding to amino acids at positions 1 to 160 in SEQ ID NO: 1. In one embodiment, the ACE3 variant has deletion of a region corresponding to amino acids at positions 1 to 200 in SEQ ID NO: 1. In one embodiment, the ACE3 variant has deletion of a region corresponding to amino acids at positions 1 to 240 in SEQ ID NO: 1.

The ACE3 variant may have deletion of a C-terminal region in addition to the DBD deletion. Examples of the C-terminal region deletion include deletion of 7 to 17 amino acids on the C-terminal, which is deletion causing improvement in protein productivity, disclosed in Non Patent Literature 5. In one embodiment, the ACE3 variant may have deletion of a region corresponding to at least 7 amino acids (from position 728 to the C-terminal) and up to 17 amino acids (from position 718 to the C-terminal) in the amino acid sequence of SEQ ID NO: 1. In one embodiment, the ACE3 variant may have deletion of one or more amino acids selected from the group consisting of amino acids corresponding to amino acids at positions -7 to -17 from the C-terminal (positions 718 to 728) in the sequence of SEQ ID NO: 1. In one embodiment, the ACE3 variant may have deletion of a region corresponding to 11 amino acids of the C-terminal (from position 724 to the C-terminal) in the amino acid sequence of SEQ ID NO: 1. In one embodiment, the ACE3 variant does not have deletion of the C-terminal of the parent ACE3.

Preferably, in the ACE3 variant, amino acids in the region except for the above-described region including the DBD and the C-terminal region are preserved.

In a preferable example, the ACE3 variant includes at least a region corresponding to amino acids at positions 280 to 701 in SEQ ID NO: 1. More preferably, the ACE3 variant includes a region corresponding to amino acids at positions 280 to 717 in SEQ ID NO: 1. More preferably, the ACE3 variant includes a region corresponding to amino acids at positions 280 to 723 in SEQ ID NO: 1. The ACE3 variant may include a region corresponding to the amino acid at positions 280 to 727 in SEQ ID NO: 1.

In a more preferable example, the ACE3 variant includes a region corresponding to amino acids at positions 260 to 701 in SEQ ID NO: 1. Further preferably, the ACE3 variant includes a region corresponding to amino acids at positions 260 to 717 in SEQ ID NO: 1. Further preferably, the ACE3 variant includes a region corresponding to amino acids at positions 260 to 723 in SEQ ID NO: 1. The ACE3 variant may include a region corresponding to amino acids at positions 260 to 727 in SEQ ID NO: 1.

In a further preferable example, the ACE3 variant includes a region corresponding to amino acids at positions 250 to 701 in SEQ ID NO: 1. Further preferably, the ACE3 variant includes a region corresponding to amino acids at positions 250 to 717 in SEQ ID NO: 1. Further preferably, the ACE3 variant includes a region corresponding to amino acids at positions 250 to 723 in SEQ ID NO: 1. The ACE3 variant may include a region corresponding to amino acids at positions 250 to 727 in SEQ ID NO: 1.

In further preferable example, the ACE3 variant includes a region corresponding to amino acids at positions 241 to 701 in SEQ ID NO: 1. Further preferably, the ACE3 variant includes a region corresponding to amino acids at positions 241 to 717 in SEQ ID NO: 1. Even preferably, the ACE3 variant includes a region corresponding to amino acids at positions 241 to 723 in SEQ ID NO: 1. The ACE3 variant may include a region corresponding to amino acids at positions 241 to 727 in SEQ ID NO: 1.

Preferably, the above-mentioned regions corresponding to the amino acid regions of SEQ ID NO: 1 each has at least 90% identity or 100% identity to any of the amino acid regions of SEQ ID NO: 1.

Alternatively, when the C-terminal region is not deleted, the ACE3 variant preferably includes a region corresponding to amino acids at positions 280 to 734 in SEQ ID NO: 1, more preferably includes a region corresponding to amino acids at positions 260 to 734 in SEQ ID NO: 1, further preferably includes a region corresponding to amino acids at positions 250 to 734 in SEQ ID NO: 1, and further preferably includes a region corresponding to amino acids at positions 241 to 734 in SEQ ID NO: 1. Preferably, the above-mentioned regions corresponding to the amino acid regions in SEQ ID NO: 1 each has at least 90% identity or 100% identity to any of the amino acid regions in SEQ ID NO: 1.

Examples of the method for modifying a parent filamentous fungus such that an ACE3 variant in which substantially the whole of the DBD and further a C-terminal region as needed are deleted (hereinafter, also referred to as target ACE3 variant) is expressed include a method of introducing an exogenous gene coding for a target ACE3 variant into a parent filamentous fungus and expressing it and a method of mutating a gene coding for ACE3 that is intrinsically included in the parent filamentous fungus into a gene coding for a target ACE3 variant.

The gene (target gene) coding for a target ACE3 variant can be synthesized by genetic engineering or chemically. For example, a target gene can be prepared by isolating a DNA of the gene (parent gene) coding for parent ACE3 from a genomic DNA of a filamentous fungus such as *Trichoderma* fungus and then deleting a part or the whole of the region coding for the DBD and further a site coding for a C-terminal region as needed. Examples of the parent gene include nucleotide sequences of SEQ ID NOs: 6 to 9 and polynucleotides consisting of sequences having at least 90% identity thereto. Alternatively, based on publicly known sequence information, a target gene in which a part or the whole of the region coding for the DBD and further a site coding for a C-terminal region are deleted can be chemically synthesized. The sequence information on ACE3 is available from the ncbi database (www.ncbi.nlm.nih.gov/]) or the like. According to the need, the target gene may be codon-optimized for the host (parent filamentous fungus) into which the gene is introduced. The information on codons that are used by various organisms is available from Codon Usage Database ([www.kazusa.or.jp/codon/]).

Examples of the method for introducing an exogenous gene coding for the target ACE3 variant into a parent filamentous fungus include a method using recombination and a method using an expression vector. For example, a given region of the genome of a parent filamentous fungus can be replaced with a target gene by homologous recombination or non-homologous recombination. Examples of the expression vector for a filamentous fungus include yeast expression vectors pNAN8142 (Biosci. Biotechnol. Biochem., 1996, 60: 383-389) and pMA91 (Biosci. Biotechnol. Biochem., 1998, 62: 1615-1618).

Examples of the method for mutating the gene coding for the ACE3 of a parent filamentous fungus include a method using recombination. For example, a parent gene in the genome of a parent filamentous fungus can be replaced with a gene (target gene) coding for a target ACE3 variant by homologous recombination or non-homologous recombination. Alternatively, a parent gene may be mutated into a target gene by replacing a DBD-coding region of the parent gene in genome with a DBD deletion fragment by homologous recombination or non-homologous recombination.

In an example of a specific method for recombination, first, a DNA construct for recombination including a target gene fragment or a DBD deletion fragment and, as needed, a drug-resistant gene or an auxotrophic gene is constructed and is introduced into a parent filamentous fungus by a common method. Subsequently, a transformant in which the construct for recombination is incorporated on the genome is selected using, for example, the drug resistance or auxotrophy as an index. As needed, it may be confirmed that the resulting transformant includes a target mutation by genomic analysis or enzyme activity analysis.

In the introduction of the DNA construct into the parent filamentous fungus, a vector that is generally used in transformation of a plasmid or the like can be used. In the introduction of the DNA construct or the vector into a cell, for example, a common method, such as a protoplast method, a protoplast PEG method, or a competent cell method, can be used. The vector for introduction of a DNA construct is not particularly limited as long as it can be stably retained and proliferate in a host cell, and examples thereof include vectors that are usually used, such as a plasmid, a cosmid, a phage, a virus, a YAC, and a BAC. Among them, a plasmid vector is preferable. Examples of the vector for introduction include pUC118.

In a preferable embodiment, the modified filamentous fungus of the present invention highly expresses a target ACE3 variant. A modified filamentous fungus that highly expresses the target ACE3 variant can be obtained by further modifying the parent filamentous fungus to improve the expression of the target ACE3 variant. Examples of the method for improving the expression of the ACE3 variant include a method for improving the transcription level of a gene (target gene) coding for the ACE3 variant. Examples of the method for improving the transcription level of a target gene include a method in which a control region (strong control region) that strongly enhances the transcription of a target gene is substituted for or inserted into the control region of the target gene on the genome of a parent filamentous fungus to operably link the strong control region to the target gene. Alternatively, the transcription level of a target gene can be improved by introducing a target gene fragment operably linked to a control region (preferably, strong control region) as needed into the genome or plasmid of a parent filamentous fungus to increase the number of the target genes that can be expressed in cells.

Examples of the control region that can be used for improving the transcription level include control regions of genes that do not decrease the transcription level even under high glucose conditions, for example, in *Trichoderma* fungi, control regions of genes, such as glyceraldehyde-3-phosphate dehydrogenase (gpd), pyruvate decarboxylase (pdc), enolase (eno), alcohol dehydrogenase (adh), triose phosphate isomerase (tpi), aldolase (fba), pyruvate kinase (pyk), citrate synthase (cit), α-ketoglutarate dehydrogenase (kdh), aldehyde dehydrogenase I (ald1), aldehyde dehydrogenase II (ald2), pyruvate dehydrogenase (pda), glucokinase (glk), actin (act1), and translation elongation factor 1α (tef1). Among them, preferable examples of the strong control region include a promoter of a pdc gene (TRIREDRAFT_121534) and a promoter of an act1 gene (TRIREDRAFT_44504).

Preferably, the expression level of a target ACE3 variant in a modified filamentous fungus of the present invention is improved compared to the expression level of ACE3 in the parent filamentous fungus. The expression level of the target ACE3 variant can be quantitatively measured as the amount of a protein or the transcription level of a gene coding for it by a publicly known method such as quantitative PCR, microarray, western blotting, ELISA, and HPLC.

Preferably, the modified filamentous fungus of the present invention further expresses XYR1 (Xylanase regulator 1). More preferably, the modified filamentous fungus of the present invention is modified so as to highly express XYR1. XYR1 is a transcriptional activator of cellulase and hemicellulase in a filamentous fungus. XYR1 has a Zn(II)₂Cys₆ binuclear cluster domain and is a main factor for xylanase gene expression regulation, and is conserved widely in Ascomycetes excluding yeasts, such as *Trichoderma* (XYR1), *Fusarium* (XYR1), *Neurospora* (XYR1), and *Aspergillus* (XLNR). XYR1 of *Trichoderma reesei* manages all of xylanase, xylose metabolism, genes and cellulase genes. The XYR1 of *Trichoderma reesei* has been registered in the ncbi database (www.ncbi.nlm.nih.gov/]) as NCBI Reference Sequence: XP_006966092.1, which is a polypeptide consisting of the amino acid sequence of SEQ ID NO: 5 encoded by the polynucleotide consisting of the nucleotide sequence of SEQ ID NO: 10.

Examples of the XYR1 that is highly expressed by the modified filamentous fungus of the present invention include a polypeptide consisting of the amino acid sequence of SEQ ID NO: 5. Other examples of the XYR1 include a polypeptide consisting of an amino acid sequence at least 90% identical to the amino acid sequence of SEQ ID NO: 5 and a polypeptide consisting of an amino acid sequence in which one to several amino acids are deleted, substituted, added, or inserted in the amino acid sequence of SEQ ID NO: 5. These polypeptides can function as transcriptional activators of cellulase and hemicellulase.

The XYR1 highly expressed by the modified filamentous fungus of the present invention may be a mutated XYR1. Preferable examples of the mutated XYR1 include mutated XYR1 in which at least one amino acid in the region corresponding to positions 810 to 833 in SEQ ID NO: 5 is mutated (i.e., substituted, deleted, inserted, or added) in a polypeptide of XYR1 (parent XYR1) consisting of the amino acid sequence of SEQ ID NO: 5 or an amino acid sequence at least 90% identical thereto. Examples of the mutated XYR1 include the mutated XYR1 disclosed in PCT/JP2020/042489.

Preferably, in the mutated XYR1, at least one amino acid selected from the group consisting of amino acids at positions corresponding to positions 817, 821, 824, 825, and 826 in SEQ ID NO: 5 is substituted in the parent XYR1.

More preferably, the mutated XYR1 consists of an amino acid sequence at least 90% identical to the amino acid sequence of SEQ ID NO: 5 and includes one or more amino acids selected from the group consisting of:
Tyr at a position corresponding to position 817 in SEQ ID NO: 5;
Lys, Phe, Trp, or Tyr, preferably Phe, at a position corresponding to position 821 in SEQ ID NO: 5;
Val, Glu, Ile, Leu, Lys, Phe, Thr, Trp, or Tyr, preferably Val, at a position corresponding to position 824 in SEQ ID NO: 5;
Tyr at a position corresponding to position 825 in SEQ ID NO: 5; and
Val, Ile, Leu, Phe, Trp, or Tyr at a position corresponding to position 826 in SEQ ID NO: 5.

More preferably, the mutated XYR1 is a mutant (V821F) of the parent XYR1 in which Val at a position corresponding to position 821 in SEQ ID NO: 5 is substituted with Phe or a mutant (A824V) of the parent XYR1 in which Ala at a position corresponding to position 824 in SEQ ID NO: 5 is substituted with Val.

As the method for highly expressing XYR1 in a modified filamentous fungus, the same method as the above-described method for highly expressing an ACE3 variant can be used. For example, the XYR1 can be highly expressed by introducing an exogenous gene coding for the XYR1 operably linked to a strong control region into a modified filamentous fungus and expressing it in the modified filamentous fungus, or operably linking a strong control region to the XYR1 gene on the genome intrinsically included in a parent filamentous fungus, or increasing the number of the XYR1 gene that can be expressed by a modified filamentous fungus.

The modified filamentous fungus of the present invention produced by the above procedure expresses an ACE3 variant in which substantially the whole of the DBD is deleted. In such a modified filamentous fungus of the present invention, the protein productivity in the absence of a cellulase inducer is improved. The modified filamentous fungus can efficiently produce a protein even in an environment in which the main carbon source is a cellulase noninducible carbon source such as glucose, for example, in the absence of a cellulase inducer. Furthermore, the modified filamentous fungus can express a cellulase-based biomass-degrading enzyme, such as cellulase and hemicellulose, even in the absence of a cellulase inducer, such as cellulose, sophorose, and cellooligosaccharide.

In a preferable embodiment, the modified filamentous fungus of the present invention expresses an ACE3 variant having the deletion of the C-terminal region in addition to the deletion of substantially the whole of the DBD. The protein productivity of the modified filamentous fungus is synergistically improved by combining the deletion of the DBD and the deletion of the C-terminal region.

In another preferable embodiment, the modified filamentous fungus of the present invention further highly expresses XYR1, in addition to the expression of an ACE3 variant in which substantially the whole of the DBD is deleted, as described above. The cellulase content in the protein produced by the modified filamentous fungus is increased by combining expressions of the ACE3 variant and XYR1, which allows efficient cellulase production.

In more preferable embodiment, the modified filamentous fungus of the present invention expresses an ACE3 variant having deletion of substantially the whole of the DBD and deletion of the C-terminal region, and further highly expresses XYR1. Such a modified filamentous fungus achieves both an improvement in the protein productivity and an increase in the cellulase content in the produced protein.

Accordingly, in a further aspect, the present invention provides a method for producing a protein using the modified filamentous fungus of the present invention described above. In the method for producing a protein by the present invention, the modified filamentous fungus of the present invention is cultured. By the culture, a target protein is produced and accumulated in the culture. The target protein can be produced by separating the target protein from the culture.

Examples of the target protein to be produced include, but not limited to, a cellulase-based biomass-degrading enzyme, such as cellulase and hemicellulase; and exoglucanase, endoglucanase, β-glucosidase, protease, lipase, mannase, arabinase, galactase, and amylase. The target protein may be one protein or a mixture of a plurality of proteins. The target protein is preferably a cellulase-based biomass-degrading enzyme, more preferably cellulase and/or hemicellulase, and further preferably cellulase and hemicellulase. Examples of the hemicellulase include xylanase, β-xylosidase, and α-arabinofuranosidase. Among them, xylanase is preferable.

Alternatively, the target protein may be a heterologous protein that is not intrinsically produced by filamentous fungi. In such a case, a recombinant filamentous fungus is produced by inserting a gene coding for the heterologous protein into the modified filamentous fungus of the present invention, and proteins including the heterologous protein can be obtained by culturing the recombinant filamentous fungus. Furthermore, secretory production of the heterologous protein in the culture is possible by operably linking a gene coding for the heterologous protein to a secretory signal peptide that functions in the filamentous fungus.

The culture medium to be used for producing the protein may be either a synthetic medium or a natural medium as long as ingredients necessary for ordinary filamentous fungal proliferation and protein production, such as a carbon source, a nitrogen source, an inorganic salt, and a vitamin, are contained.

The carbon source may be any carbon source that can be utilized by the modified filamentous fungus, and examples thereof include carbohydrates, such as glucose and fructose; sugar alcohols, such as sorbitol; alcohols, such as ethanol and glycerol; and organic acids, such as acetic acid. These carbon sources may be used alone or in combination of two or more thereof.

Preferably, in the method for producing a protein according to the present invention, the modified filamentous fungus is cultured in an environment where a cellulase noninducible carbon source is a main carbon source. Examples of the cellulase noninducible carbon source include glucose, fructose, sucrose, maltose, and glycerol. Among them, glucose is preferable in terms of cost. When the target protein to be produced is a cellulase-based biomass-degrading enzyme, the culture by this method may be performed in the presence of a cellulase inducer, such as cellulose, sophorose, and cellooligosaccharide, and an enhanced production of the target protein is possible even in the absence of the inducer, and the culture is not limited to the use or non-use of an inducer. In addition, since the present invention efficiently produces a protein such as a cellulase-based biomass-degrading enzyme while further reducing the catabolite repression, the modified filamentous fungus may be cultured while feeding a noninducible carbon source such as glucose to the culture. On this occasion, it is preferable to dissolve the cellulase noninducible carbon source, for example, glucose, in ammonia water or an aqueous solution containing an ammonium salt serving as a nitrogen source and to perform culture while feeding the solution to the culture, in terms of culture efficiency and suppression of foaming during culturing.

Examples of the nitrogen source include ammonia, an ammonium salt such as ammonium sulfate, a nitrogen compound such as amine, and a natural nitrogen source such as peptone and soybean hydrolysate.

Examples of the inorganic salt include potassium phosphate, magnesium sulfate, sodium chloride, ferrous sulfate, and potassium carbonate.

Examples of the vitamin include biotin and thiamine. Furthermore, a substance required for growth of the modified filamentous fungus of the present invention can be added as needed.

The culture is preferably performed in aerobic conditions such as shaking culture and aerated and agitated culture. The culture temperature is preferably 10°C or more, more preferably 20°C or more, and more preferably 25°C or more and preferably 50°C or less, more preferably 42°C or less, and more preferably 35°C or less, and is preferably from 10°C to 50°C, more preferably from 20°C to 42°C, and more preferably from 25°C to 35°C. The pH during culturing is from 3 to 9 and preferably from 4 to 5. The culture time is from 10 hours to 10 days and preferably from 2 to 7 days.

After the culture, the target protein is separated from the resulting culture by a common method. For example, the target protein can be separated from the culture by collecting the culture, performing cell disruption treatment, such as ultrasonication or pressurization as needed, and performing an appropriate combination of filtration, centrifugation, ultrafiltration, salting out, dialysis, chromatography, and the like. The degree of separation of the target protein is not particularly limited. For example, the culture supernatant or its roughly separated and purified product can be obtained as a composition containing the target protein.

The present invention also encompasses the following materials, producing methods, uses, methods, and so on as exemplary embodiments. However, the present invention is not limited to these embodiments.

[1] A modified filamentous fungus, which expresses an ACE3 variant, wherein
   the ACE3 variant is a variant in which substantially the whole of a Zn(II)₂Cys₆-type DNA-binding domain (DBD) of ACE3 is deleted.
[2] The modified filamentous fungus according to [1], wherein the ACE3 is preferably a polypeptide consisting of an amino acid sequence of any one of SEQ ID NOs: 1 to 4 or an amino acid sequence having at least 90% identity thereto.
[3] The modified filamentous fungus according to [2], wherein the Zn(II)₂Cys₆-type DNA-binding domain (DBD) is preferably a region corresponding to amino acids at positions 120 to 160 in SEQ ID NO: 1.
[4] The modified filamentous fungus according to [2], wherein the ACE3 variant is preferably any one of the following (1) to (4):
   (1) a variant in which a part or the whole of C2 and a part or the whole of C4 in the DBD is deleted, where the C2 is a region corresponding to positions 120 to 131 in SEQ ID NO: 1, the C4 is a region corresponding to positions 132 to 160 in SEQ ID NO: 1, and a part of the C4 is preferably a region including at least two cysteines in C4, more preferably a region including at least three cysteines in C4, further preferably a region including four cysteines in C4, and further preferably a region corresponding to amino acids at positions 132 to 151 in SEQ ID NO: 1;
   (2) a variant in which 80% or more, preferably 90% or more, and more preferably 95% or more of the amino acid sequence of a region corresponding to amino acids at positions 120 to 160 in SEQ ID NO: 1 is deleted, and six cysteines in the region are deleted;
   (3) a variant in which a region corresponding to amino acids at positions 120 to 151 in SEQ ID NO: 1 is deleted; and
   (4) a variant in which a region corresponding to amino acids at positions 1 to 151 in SEQ ID NO: 1 is deleted, or
   the ACE3 variant is more preferably any one of the following (5) to (7):
   (5) a variant in which a region corresponding to amino acids at positions 1 to 160 in SEQ ID NO: 1 is deleted;
   (6) a variant in which a region corresponding to amino acids at positions 1 to 200 in SEQ ID NO: 1 is deleted; and
   (7) a variant in which a region corresponding to amino acids at positions 1 to 240 in SEQ ID NO: 1 is deleted.
[5] The modified filamentous fungus according to any one of [2] to [4], wherein in the ACE3 variant:
   preferably, a region corresponding to at least 7 amino acids and up to 17 amino acids on the C-terminal in the amino acid sequence of SEQ ID NO: 1 is deleted;
   preferably, one or more amino acids selected from the group consisting of the amino acids corresponding to amino acids at positions -7 to -17 from the C-terminal (positions 718 to 728) in the sequence of SEQ ID NO: 1 are deleted; or
   more preferably, a region corresponding to 11 amino acids on the C-terminal in the amino acid sequence of SEQ ID NO: 1 is deleted.
[6] The modified filamentous fungus according to any one of [2] to [5], wherein the ACE3 variant includes:
   preferably a region corresponding to amino acids at positions 280 to 701 in SEQ ID NO: 1;
   preferably a region corresponding to amino acids at positions 280 to 717 in SEQ ID NO: 1;
   preferably a region corresponding to amino acids at positions 280 to 723 in SEQ ID NO: 1;
   preferably a region corresponding to amino acids at positions 280 to 727 in SEQ ID NO: 1;
   preferably a region corresponding to amino acids at positions 260 to 701 in SEQ ID NO: 1;
   preferably a region corresponding to amino acids at positions 260 to 717 in SEQ ID NO: 1;
   preferably a region corresponding to amino acids at positions 260 to 723 in SEQ ID NO: 1;
   preferably a region corresponding to amino acids at positions 260 to 727 in SEQ ID NO: 1;
   preferably a region corresponding to amino acids at positions 250 to 701 in SEQ ID NO: 1;
   preferably a region corresponding to amino acids at positions 250 to 717 in SEQ ID NO: 1;
   preferably a region corresponding to amino acids at positions 250 to 723 in SEQ ID NO: 1;
   preferably a region corresponding to amino acids at positions 250 to 727 in SEQ ID NO: 1;
   preferably a region corresponding to amino acids at positions 241 to 701 in SEQ ID NO: 1;
   preferably a region corresponding to amino acids at positions 241 to 717 in SEQ ID NO: 1;
   preferably a region corresponding to amino acids at positions 241 to 723 in SEQ ID NO: 1; or
   preferably a region corresponding to amino acids at positions 241 to 727 in SEQ ID NO: 1.
[7] The modified filamentous fungus according to any one of [1] to [6], wherein a gene that expresses the ACE3 variant preferably has been introduced into the modified filamentous fungus.
[8] The modified filamentous fungus according to [7], wherein
   the gene that expresses the ACE3 variant is preferably operably linked to a control region that promotes transcription of the gene, and
   the control region is preferably selected from the group consisting of pdc promoter and act1 promoter.
[9] The modified filamentous fungus according to any one of [1] to [8], wherein the filamentous fungus is:
   preferably a *Trichoderma* fungus; and
   more preferably *Trichoderma reesei* or its mutant strain.
[10] The modified filamentous fungus according to any one of [1] to [9], wherein
   the modified filamentous fungus preferably expresses XYR1 or a mutated XYR1, more preferably a mutated XYR1,
   preferably, the XYR1 or a mutated XYR1 is a polypeptide consisting of the amino acid sequence of SEQ ID NO: 5 or an amino acid sequence having at least 90% identity thereto,
   preferably, in the mutated XYR1, at least one amino acid in a region corresponding to positions 810 to 833 in SEQ ID NO: 5 is mutated in the amino acid sequence of SEQ ID NO: 5 or an amino acid sequence having at least 90% identity thereto,
   more preferably, in the mutated XYR1, at least one amino acid selected from the group consisting of the amino acids at positions corresponding to positions 817, 821, 824, 825, and 826 in SEQ ID NO: 5 is substituted in the amino acid sequence of SEQ ID NO: 5 or an amino acid sequence having at least 90% identity thereto, and
   further preferably, the mutated XYR1 consists of an amino acid sequence having at least 90% identity to the amino acid sequence of SEQ ID NO: 5 and includes one or more amino acids selected from the group consisting of the followings:
      Tyr at a position corresponding to position 817 in SEQ ID NO: 5;
      Lys, Phe, Trp, or Tyr, preferably Phe, at a position corresponding to position 821 in SEQ ID NO: 5;
      Val, Glu, Ile, Leu, Lys, Phe, Thr, Trp, or Tyr, preferably Val, at a position corresponding to position 824 in SEQ ID NO: 5;
      Tyr at a position corresponding to position 825 in SEQ ID NO: 5; and
      Val, Ile, Leu, Phe, Trp, or Tyr at a position corresponding to position 826 in SEQ ID NO: 5.
[11] A method for producing a protein, comprising: culturing the modified filamentous fungus according to any one of [1] to [10].
[12] The method according to [11], wherein the protein is preferably cellulase and/or hemicellulase.
[13] The method according to [11] or [12], wherein the culturing is preferably performed in the presence of glucose.
[14] The method according to [13], wherein the culturing is preferably performed in the absence of a cellulase inducer.
[15] A method for producing a modified filamentous fungus, comprising:
   modifying a parent filamentous fungus so as to express an ACE3 variant, wherein
   the ACE3 variant is a variant in which substantially the whole of a Zn(II)₂Cys₆-type DNA-binding domain (DBD) of ACE3 is deleted.
[16] The method according to [15], wherein the ACE3 is preferably a polypeptide consisting of the amino acid sequence of SEQ ID NO: 1 or an amino acid sequence having at least 90% identity thereto.
[17] The method according to [16], wherein the Zn(II)₂Cys₆-type DNA-binding domain (DBD) is preferably a region corresponding to amino acids at positions 120 to 160 in SEQ ID NO: 1.
[18] The method according to [16], wherein the ACE3 variant is preferably any one of the following (1) to (4) :
   (1) a variant in which a part or the whole of C2 and a part or the whole of C4 in the DBD is deleted, where the C2 is a region corresponding to positions 120 to 131 in SEQ ID NO: 1, the C4 is a region corresponding to positions 132 to 160 in SEQ ID NO: 1, and a part of the C4 is preferably a region including at least two cysteines in C4, more preferably a region including at least three cysteines in C4, further preferably a region including four cysteines in C4, and further preferably a region corresponding to amino acids at positions 132 to 151 in SEQ ID NO: 1;
   (2) a variant in which 80% or more, preferably 90% or more, and more preferably 95% or more of the amino acid sequence of a region corresponding to amino acids at positions 120 to 160 in SEQ ID NO: 1 are deleted, and six cysteines in the region are deleted;
   (3) a variant in which a region corresponding to amino acids at positions 120 to 151 in SEQ ID NO: 1 is deleted; and
   (4) a variant in which a region corresponding to amino acids at positions 1 to 151 in SEQ ID NO: 1 is deleted, or
   the ACE3 variant is more preferably any one of the following (5) to (7):
   (5) a variant in which a region corresponding to amino acids at positions 1 to 160 in SEQ ID NO: 1 is deleted;
   (6) a variant in which a region corresponding to amino acids at positions 1 to 200 in SEQ ID NO: 1 is deleted; and
   (7) a variant in which a region corresponding to amino acids at positions 1 to 240 in SEQ ID NO: 1 is deleted.
[19] The method according to any one of [16] to [18], wherein
   in the ACE3 variant,
   preferably, a region corresponding to at least 7 amino acids and up to 17 amino acids on the C-terminal in the amino acid sequence of SEQ ID NO: 1 is deleted;
   preferably, one or more amino acids selected from the group consisting of amino acids corresponding to amino acids at positions -7 to -17 from the C-terminal (position 718 to position 728) in the sequence of SEQ ID NO: 1 are deleted; or
   more preferably, a region corresponding to 11 amino acids on the C-terminal in the amino acid sequence of SEQ ID NO: 1 is deleted.
[20] The method according to any one of [16] to [19], wherein the ACE3 variant includes:
   preferably, a region corresponding to amino acids at positions 280 to 701 in SEQ ID NO: 1;
   preferably, a region corresponding to amino acids at positions 280 to 717 in SEQ ID NO: 1;
   preferably, a region corresponding to amino acids at positions 280 to 723 in SEQ ID NO: 1;
   preferably, a region corresponding to amino acids at positions 280 to 727 in SEQ ID NO: 1;
   preferably, a region corresponding to amino acids at positions 260 to 701 in SEQ ID NO: 1;
   preferably, a region corresponding to amino acids at positions 260 to 717 in SEQ ID NO: 1;
   preferably, a region corresponding to amino acids at positions 260 to 723 in SEQ ID NO: 1;
   preferably, a region corresponding to amino acids at positions 260 to 727 in SEQ ID NO: 1;
   preferably, a region corresponding to amino acids at positions 250 to 701 in SEQ ID NO: 1;
   preferably, a region corresponding to amino acids at positions 250 to 717 in SEQ ID NO: 1;
   preferably, a region corresponding to amino acids at positions 250 to 723 in SEQ ID NO: 1;
   preferably, a region corresponding to amino acids at positions 250 to 727 in SEQ ID NO: 1;
   preferably, a region corresponding to amino acids at positions 241 to 701 in SEQ ID NO: 1;
   preferably, a region corresponding to amino acids at positions 241 to 717 in SEQ ID NO: 1;
   preferably, a region corresponding to amino acids at positions 241 to 723 in SEQ ID NO: 1; or
   preferably, a region corresponding to amino acids at positions 241 to 727 in SEQ ID NO: 1.
[21] The method according to any one of [15] to [20], wherein the modification of the parent filamentous fungus preferably includes introduction of a gene that expresses the ACE3 variant into the parent filamentous fungus.
[22] The method according to [21], wherein
   the gene that expresses the ACE3 variant is preferably operably linked to a control region that promotes transcription of the gene, and
   the control region is preferably selected from the group consisting of pdc promoter and act1 promoter.
[23] The method according to any one of [15] to [22], wherein the filamentous fungus is:
   preferably a *Trichoderma* fungus; and
   more preferably *Trichoderma reesei* or its mutant strain.

### Examples

The present invention will now be further specifically described using examples.

### Example 1 Construction of plasmid DNA for gene introduction

The following DNA fragments 1 to 5 were prepared by PCR using a genomic DNA of *Trichoderma reesei* (*T. reesei*) as a template. Fragment 1: a promotor region of about 1.5 kbp upstream of act1 gene (TRIREDRAFT_44504); Fragment 2: a polynucleotide (SEQ ID NO: 6, about 2.9 kbp) coding for the full-length polypeptide of ACE3 (SEQ ID NO: 1), Fragment 3: a polynucleotide (SEQ ID NO: 7, about 2.0 kbp) coding for the polypeptide (SEQ ID NO: 2) of ACE3 with partial DBD deletion, Fragment 4: a polynucleotide (SEQ ID NO: 10, about 3.0 kbp) coding for the full-length polypeptide of XYR1 (SEQ ID NO: 5), Fragment 5: a terminator region of about 0.6 kbp downstream of cbh1 gene (TRIREDRAFT_44504), and Fragment 6: a region of about 2.7 kbp of pyr4 gene (TRIREDRAFT_74020).

The fragments 1 and 2 were linked to construct a cassette 1: Pact1-TrACE3 (1-734). The fragments 1 and 3 were linked to construct a cassette 2: Pact1-TrACE3 (1-629). The fragments 1 and 4 were linked to construct a cassette 3: Pact1-XYR1. A fragment 7 of about 0.5 kbp and a fragment 8 of about 1.0 kbp were disposed upstream and downstream, respectively, of the fragment 6 as homologous sequences for pop-out to prepare a transformation marker fragment. The fragment 5 and the transformation marker fragment were linked to construct a cassette 4: Tcbh1-pyr4.

An about 1.5 kbp region (fragment 9) upstream and an about 1.4 kbp region (fragment 10) downstream of the reel gene (TRIREDRAFT_72611) were inserted into the HincII restriction enzyme breakpoint of pUC118 (Takara Bio Inc.) while adding a smiI restriction enzyme site to construct a cassette 5: ΔRCE1. An about 1.6 kbp region (fragment 11) upstream and an about 1.2 kbp region (fragment 12) downstream of the ace1 gene (TRIREDRAFT_75418) were inserted into the HincII restriction enzyme breakpoint of pUC118 (Takara Bio Inc.) while adding a smiI restriction enzyme site to construct a cassette 6: ΔACE1.

The linking of DNA fragments was performed according to the protocol of In-Fusion HD Cloning Kit (Takara Bio Inc.). The constructed cassettes, the DNA fragments contained therein, and the primers used for constructing the cassettes are shown in Table 1.

**[Table 1]**

| Cassette | | | | Primers (5'-3' sequences) | SEQ ID NO: |
|---|---|---|---|---|---|
| Cassette 1 Pact1-TrACE (1-734) | Fragment 1 | About 1.5 kbp | Fw | ACGGGGTACGCGTGAAATTG | 11 |
| | | | Rv | TGTGACTGATTAATGTATGA | 12 |
| | Fragment 2 | About 2.9 kbp | Fw | TAATCAGTCACAATGGCCACAGCGGCCGCGGCAGC; | 13 |
| | | | Rv | TTTCGCCACGGAGCTTTAGCCAACAACGGTAGTGG | 14 |
| Cassette 2 Pact1-TrACES (1-629) | Fragment 1 | About 1.5 kbp | Fw | ACGGGGTACGCGTGAAATTG | 11 |
| | | | Rv | TGTGACTGATTAATGTATGA | 12 |
| | Fragment 3 | About 2.9 kbp | Fw | CATTAATCAGTCACAATGCTGCGCTACTCCCCCGT | : 15 |
| | | | Rv | TTTCGCCACGGAGCTTTAGCCAACAACGGTAGTGG | 14 |
| Cassette 3 act1-XYR1 | Fragment 1 | About 1.5 kbp | Fw | ACGGGGTACGCGTGAAATTG | , 11 |
| | | | Rv | TGTGACTGATTAATGTATGA | 12 |
| | Fragment 4 | About 3.0 kbp | Fw | CATTAATCAGTCACAATGTTGTCCAATCCTCTCCG | 16 |
| | | | Rv | TTTCGCCACGGAGCTTTAGAGGGCCAGACCGGTTC | 17 |
| Cassette 4 Tcbh1-pyr4 | Fragment 5 | About 0.6 kbp | Fw | AGCTCCGTGGCGAAAGCCTG | 18 |
| | | | Rv | CTCGGCTACGTTGTCATCGT | 19 |
| | Fragment 7 | About 0.5 kbp | Fw | GACAACGTAGCCGAGAAGTACCGCGCGCTTGACAA | 20 |
| | | | Rv | ACCTTGGCTGGTTTGCTGAATGCCCGGTGGTAAGC | 21 |
| | Fragment 6 | About 2.7 kbp | Fw | CAAACCAGCCAAGGTAGGTA | 22 |
| | | | Rv | AAGCGCGCGGTACTTCCATCACATGTCAATGTCAC | 23 |
| | Fragment 8 | About 1.0 kbp | Fw | AAGTACCGCGCGCTTGACAA | 24 |
| | | | Rv | TTGGTTCTTGGTTTGGAGGG | 25 |
| Cassette 5 ΔRCE1 | Fragment 9 | About 1.5 kbp | Fw | CTAGAGTATTTAAATGGTCAACGGAGGCCAGAAGA | 26 |
| | | | Rv | GACTGCCTGCCTCCCAGTTT | 27 |
| | Fragment 10 | About 1.4 kbp | Fw | CATAACTGAGAGAACCAGAA | 28 |
| | | | Rv | TGCAGGTATTTAAATATTTTGCGCATGACGGGCGA | 29 |
| Cassette 6 ΔACE1 | Fragment 11 | About 1.6 kbp | Fw | ATTTAAATTAACCTGATTTCAAC | 30 |
| | | | Rv | GGCGGCCGAGATCTGTGTTC | 31 |
| | Fragment 12 | About 1.2 kbp | Fw | AAAGATTGCGACACATACAA | 32 |
| | | | Rv | ATTTAAATAGAGGTAGTCTAGTC | 33 |

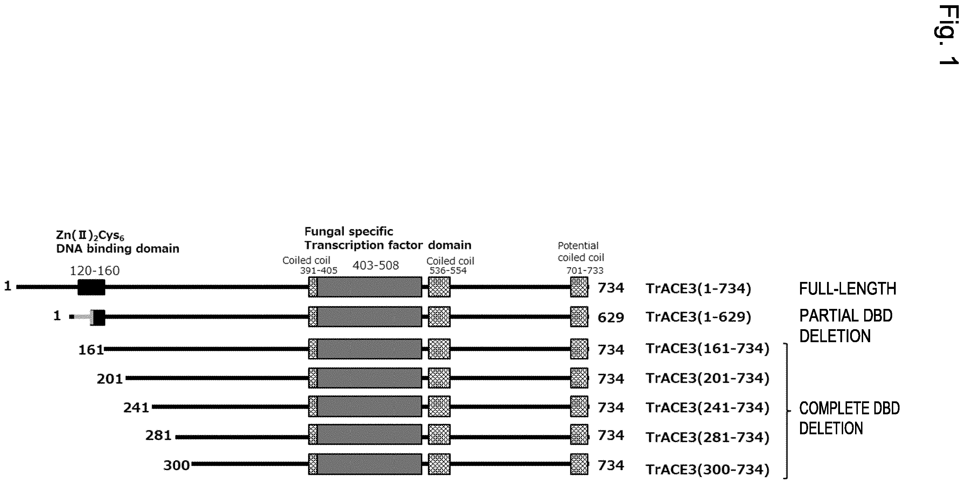

The cassettes 1 and 4 were linked by PCR using the primers shown in Table 2 and inserted between an upstream region and a downstream region of the reel gene of the cassette 5 to construct a full-length ace3 constitutive expression plasmid pUC-Pact1-TrACE3 (1-734) (plasmid 1). The cassettes 2 and 4 were linked by PCR using the primers shown in Table 2 and inserted between an upstream region and a downstream region of the rce1 gene of the cassette 5 to construct a partial DBD deletion ace3 constitutive expression plasmid pUC-Pact1-TrACE3 (1-629) (plasmid 2). The plasmid 1 expresses the full-length ACE3 of *T. reesei* consisting of the sequence of SEQ ID NO: 1, and the plasmid 2 expresses an ACE3 variant in which a part (C2) of the DBA was deleted in the sequence of SEQ ID NO: 1 (Figure 1).

The cassettes 3 and 4 were linked by PCR using the primers shown in Table 2 and inserted between an upstream region and a downstream region of the ace1 gene of the cassette 6 to construct an xyr1 gene constitutive expression plasmid pUC-Pact1-XYR1 (plasmid 3). The plasmid 3 expresses xylanase XYR1.

By PCR using the primers shown in Table 3 and the plasmid 1 as a template, pUC-Pact1-TrACE3 (161-734) (plasmid 4), pUC-Pact1-TrACE3 (201-734) (plasmid 5), pUC-Pact1-TrACE3 (241-734) (plasmid 6), pUC-Pact1-TrACE3 (281-734) (plasmid 7), and pUC-Pact1-TrACE3 (300-734) (plasmid 8) were respectively constructed. The plasmids 4 to 8 express ACE3 variants in which prescribed regions including the DBD on the N-terminal side were deleted in the sequence of SEQ ID NO: 1 (Figure 1).

A polynucleotide (SEQ ID NO: 8, about 2.0 kbp) coding for a variant (SEQ ID NO: 3) of the ACE3 polypeptide of *Trichoderma atroviride* and a polynucleotide (SEQ ID NO: 9, about 2.0 kbp) coding for a variant (SEQ ID NO: 4) of the ACE3 polypeptide of *Trichoderma harzianum* were artificially synthesized. The obtained fragments were linked to the plasmid 1 by PCR using the primers shown in Table 4 to construct pUC-Pact1-TaACE3 (1-630) (plasmid 9) and pUC-Pact1-ThACE3 (1-630) (plasmid 10), respectively. The plasmids 9 and 10 express ACE3 variants in which a part (C2) of the DBD was deleted.

By PCR using the primers shown in Table 4 and the plasmid 9 or 10 as a template, pUC-Pact1-TaACE3 (137-630) (plasmid 11) and pUC-Pact1-ThACE3 (137-630) (plasmid 12) were respectively constructed. The plasmids 11 and 12 express complete DBD deletion ACE3 variants in which 136 amino acids on the N-terminal side were further deleted in the ACE3 variants of SEQ ID NO: 3 and SEQ ID NO: 4, respectively.

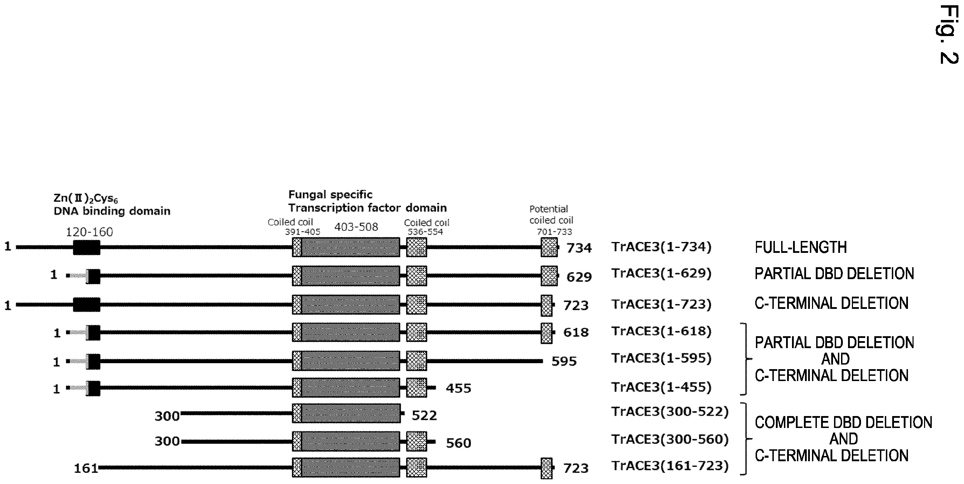

A plasmid (plasmid 13) that expresses an ACE3 variant in which a prescribed region on the C-terminal side was deleted in ACE3 (SEQ ID NO: 1) of *T. reesei* was constructed by PCR using the primers shown in Table 5 and the plasmid 1 as a template. Plasmids (plasmids 14 to 17) that express ACE3 variants in which prescribed regions on the N-terminal side and the C-terminal side in ACE3 (SEQ ID NO: 1) of *T. reesei* were deleted were constructed by PCR using the primers shown in Table 5 and the plasmid 2 or 4 as a template. Plasmids (plasmids 18 and 19) that express ACE3 variants in which prescribed regions on the N-terminal side and the C-terminal side in ACE3 (SEQ ID NO: 1) of *T. reesei* were deleted were constructed by two-step PCR using the primers shown in Table 5 and the plasmid 1 as a template. The configurations of ACE3 variants included in the plasmids 13 to 18 are shown in Figure 2.

By PCR using the primers shown in Table 5 and plasmid 3 as a template, pUC-Pact1-XYR1 (V821F) (plasmid 20) was constructed. This is a plasmid coding for mutated XYR1 (V821F) having amino acid substitution, V821F, in the amino acid sequence of SEQ ID NO: 5.

The plasmids 1 to 20 constructed in this Example, fragments (cassettes, plasmids, and the like.) included therein and primers used for construction thereof, and ACE3 expressed thereby are shown in Tables 2 to 5.

**[Table 2]**

| Plasmid | | | Primers (5'-3' sequences) | | SEQ ID NO: | ACE3 | |
|---|---|---|---|---|---|---|---|
| No. | Name | Contained fragment | | | | | |
| 1 | pUC-Pact1-TrACE3 (1-734) | Cassette 5 | Fw | TCACGCGTACCCCGTGACTGCCTGCCTCCCAGTTT | 34 | TrACE3 (1-734) (SEQ ID NO:1) | T.reesei full-length ACE3 |
| | | | Rv | CAAACCAAGAACCAACATAACTGAGAGAACCAGAA | 35 | | |
| | | Cassette 1 | Fw | ACGGGGTACGCGTGAAATTG | 11 | | |
| | | | Rv | CGCCACGGAGCTTTAGCCAACAACGGTAGTGGACG | 36 | | |
| | | Cassette 4 | Fw | AGCTCCGTGGCGAAAGCCTG | 18 | | |
| | | | Rv | TTGGTTCTTGGTTTGGAGGG | 25 | | |
| 2 | pUC-Pact1-TrACE3 (1-629) | Cassette 5 | Fw | TCACGCGTACCCCGTGACTGCCTGCCTCCCAGTTT | 34 | TrACE3 (1-629) (SEQ ID NO:2) Partial DBD-deleted variant of T.reesei ACE3 | |
| | | | Rv | CAAACCAAGAACCAACATAACTGAGAGAACCAGAA | 35 | | |
| | | Cassette 2 | Fw | ACGGGGTACGCGTGAAATTG | 11 | | |
| | | | Rv | CGCCACGGAGCTTTAGCCAACAACGGTAGTGGACG | 36 | | |
| | | Cassette 4 | Fw | AGCTCCGTGGCGAAAGCCTG | 18 | | |
| | | | Rv | TTGGTTCTTGGTTTGGAGGG | 25 | | |
| 3 | pUC-Pact1-XYR1 | Cassette 6 | Fw | CAAACCAAGAACCAAAAAGATTGCGACACATACAA | 37 | - | - |
| | | | Rv | GGCGGCCGAGATCTGTGTTC | 31 | | |
| | | Cassette 3 | Fw | CAGATCTCGGCCGCCACGGGGTACGCGTGAAATTG | 38 | | |
| | | | Rv | TTTCGCCACGGAGCTTTAGAGGGCCAGACCGGTTC | 17 | | |
| | | Cassette 4 | Fw | AGCTCCGTGGCGAAAGCCTG | 18 | | |
| | | | Rv | TTGGTTCTTGGTTTGGAGGG | 25 | | |

**[Table 3]**

| Plasmid | | | Primers (5'-3' sequences) | | SEQ ID NO: | ACE3 | |
|---|---|---|---|---|---|---|---|
| No. | Name | Contained fragment | | | | | |
| 4 | pUC-Pact1-TrACE3 (161-734) | Plasmid 1 | Fw | TAATCAGTCACAATGGGCCCCAAAGCGAGGAAGAA | 39 | TrACE3 (161-734) | Complete DBD-deleted variant of T.reesei ACE3 |
| | | | Rv | CATTGTGACTGATTAATGTATGA | 40 | | |
| 5 | pUC-Pact1-TrACE3 (201-734) | Plasmid 1 | Fw | TAATCAGTCACAATGGTAGCCGCGCTGCAGCCCTT | 41 | TrACE3 (201-734) | |
| | | | Rv | CATTGTGACTGATTAATGTATGA | 40 | | |
| 6 | pUC-Pact1-TrACE3 (241-734) | Plasmid 1 | Fw | TAATCAGTCACAATGCTCTCCACCATCCAGAACAT : | 42 | TrACE3 (281-734) | |
| | | | Rv | CATTGTGACTGATTAATGTATGA | 40 | | |
| 7 | pUC-Pact1-TrACE3 (281-734) | Plasmid 1 | Fw | TAATCAGTCACAATGTACCTCTACCCCCTCACCCC | 43 | TrACE3 (241-734) | |
| | | | Rv | CATTGTGACTGATTAATGTATGA : | 40 | | |
| 8 | pUC-Pact1-TrACE3 (300-734) | Plasmid 1 | Fw | TAATCAGTCACAATGTACATCTTCTCCCAGCCCTT | 44 | TrACE3 (300-734) | |
| | | | Rv | CATTGTGACTGATTAATGTATGA : | 40 | | |

**[Table 4]**

| Plasmid | | | Primers (5'-3' sequences) | | SEQ ID NO: | ACE3 | |
|---|---|---|---|---|---|---|---|
| No. | Name | Contained fragment | | | | | |
| 9 | pUC-Pact1-TaACE3 (1-630) | Plasmid 1 | Fw | AGCTCCGTGGCGAAAGCCTG | 18 | TaACE3 (1-630) | (SEQ ID NO:3) Partial DBD deletion variant of T.atroviridei ACE3 |
| | | | Rv | TGTGACTGATTAATGTATGA | 12 | | |
| | | T. atroviride ACE3 (synthetic) | Fw | CATTAATCAGTCACAATGCTGCGCTACGCCGTCCA: | 45 | | |
| | | | Rv | TTTCGCCACGGAGCTTTAGCCAACAACGGTATTGG | 46 | | |
| 10 | pUC-Pact1-ThACE3 (1-630) | Plasmid 1 | Fw | AGCTCCGTGGCGAAAGCCTG | 18 | ThACE3 (1-630) (SEQ ID NO:4) | Partial DBD deletion variant of T.harzianumi ACE3 |
| | | | Rv | TGTGACTGATTAATGTATGA | 12 | | |
| | | T. harzianum ACE3 (synthetic) | Fw | TAATCAGTCACAATGCTGCGCTACTCCTCCGTCCC | 47 | | |
| | | | Rv | TTTCGCCACGGAGCTTTAGCCAACAACGGTATTAG | 48 | | |
| 11 | pUC-Pact1-TaACE3 (137-630) | Plasmid 9 | Fw | TAATCAGTCACAATGCTCTCCACCATCCAAAACAT | 49 | TaACE3 (137-630) | Complete DBD deletion variant of T.atroviridei ACE3 |
| | | | Rv | CATTGTGACTGATTAATGTATGA | 40 | | |
| 12 | pUC-Pact1-ThACE3 (137-630) | Plasmid 10 | Fw | TAATCAGTCACAATGCTCTCCACAATTCAGAACAT | 50 | ThACE3 (137-630) | Complete DBD deletion variant of T.harzianumi ACE3 |
| | | | Rv | CATTGTGACTGATTAATGTATGA | 40 | | |

**[Table 5]**

| Plasmid | | | Primers (5'-3' sequences) | | SEQ ID NO: | ACE3 | |
|---|---|---|---|---|---|---|---|
| No. | Name | Contained fragment | | | | | |
| 13 | pUC-Pact1- TrACE3 (1-723) | Plasmid 1 | Fw | TAAAGCTCCGTGGCGAAAGCCTG | 51 | TrACE3 (1-723) | C-terminal deletion variant of T. reesei ACE3 |
| | | | Rv | CGCCACGGAGCTTTAGTCCGACGCCTTCGAGTCCA | 52 | | |
| 14 | pUC-Pact1- TrACE3 (1-618) | Plasmid 2 | Fw | TAAAGCTCCGTGGCGAAAGCCTG | 51 | TrACE3 (1-618) | Partial DBD and C-terminal deletion variant of T. reesei ACE3 |
| | | | Rv | CGCCACGGAGCTTTAGTCCGACGCCTTCGAGTCCA : | 52 | | |
| 15 | pUC-Pact1- TrACE3 (161-723) | Plasmid 4 | Fw | TAAAGCTCCGTGGCGAAAGCCTG | 51 | TrACE3 | DBD complete and C-terminal deletion variant of T.reesei ACE3 |
| | | | Rv | CGCCACGGAGCTTTAGTCCGACGCCTTCGAGTCCA | 52 | | |
| 16 | pUC-Pact1- TrACE3 (1-595) | Plasmid 2 | Fw | TAAAGCTCCGTGGCGAAAGCCTG | 51 | TrACE3 (1-595) | Partial DBD and C-terminal deletion variant of T. reesei ACE3 |
| | | | Rv | CGCCACGGAGCTTTAAGCCAAGGGTGAATCCTGGT | 53 | | |
| 17 | pUC-Pact1-TrACE3 (1-455) | Plasmid 2 | Fw | TAAAGCTCCGTGGCGAAAGCCTG | 51 | TrACE3 | Partial DBD and C side deletion variant of T. reesei ACE3 |
| | | | Rv | CGCCACGGAGCTTTAGTTCGGGGGCATGGTCCCTC | 54 | | |
| 18 | pUC-Pact1- TrACE3 (300-522) | Plasmid 1 | Fw | TAAAGCTCCGTGGCGAAAGCCTG | 51 | TrACE3 | (300-522) Complete DBD and C side deletion variant of T.reesei ACE3 |
| | | | Rv | CATTGTGACTGATTAATGTATGA | 40 | | |
| | | Plasmid 1 | Fw | TAATCAGTCACAATGTACATCTTCTCCCAGCCCTT | 44 | | |
| | | | Rv | CGCCACGGAGCTTTATATGAAGCTCTTCCGGGGCG | 55 | | |
| 19 | pUC-Pact1- TrACE3 (300-560) | Plasmid 1 | Fw | TAAAGCTCCGTGGCGAAAGCCTG | 51 | TrACE3 | Complete DBD and C side deletion variant of T. reesei ACE3 |
| | | | Rv | CATTGTGACTGATTAATGTATGA | 40 | | |
| | | Plasmid 1 | Fw | TAATCAGTCACAATGTACATCTTCTCCCAGCCCTT | 44 | | |
| | | | Rv | CGCCACGGAGCTTTAGTTCGGGGGCATGGTCCCTC | 54 | | |
| 20 | pUC-Pact1- XYR1 (V821F) | Plasmid 3 | Fw | CACGCGTTCTCGGCTGCCGAAGCTATT | 56 | - | - |
| | | | Rv | AGCCGAGAACGCGTGGCTCGTCGCCGT | 57 | | |

The constructed plasmids were replicated. The plasmids produced above were introduced into competent cells Escherichia coli DH5α Competent Cells (Takara Bio Inc.), and the cells were cultured in an LB medium containing ampicillin (37°C, 1 day). The plasmids were collected and purified from the cultured cells using NucleoSpin(TM) Plasmid (Macherey-Nagel GmbH & Co. KG).

### Example 2 Production of modified filamentous fungus

*Trichoderma reesei* E1AB1 (JN13) pyr4 gene deletion strain (JN13Δpyr4 strain) was transformed by introduction of a DNA fragment derived from the plasmid constructed in Example 1. The plasmid constructed in Example 1 was linearized by cleavage at the smiI restriction enzyme site and introduced into a parent strain by a protoplast PEG method (Biotechnol. Bioeng, 2012, 109 (1): 92-99) for transformation. The transformant was selected using the pyr4 gene as a marker with a selection medium (2% glucose, 1.1 M sorbitol, 2% agar, 0.2% KH₂PO₄ (pH 5.5), 0.06% CaCl₂·2H₂O, 0.06% CsCl₂, 0.06% MgSO₄·7H₂O, 0.5% (NH₄)₂SO₄, 0.1% Trace element 1; % is w/v% in every ingredient). The composition of the trace element 1 was as follows: 0.5 g of FeSO₄·7H₂O, 0.2 g of CoCl₂, 0.16 g of MnSO₄·H₂O, and 0.14 g of ZnSO₄·7H₂O were diluted up to 100 mL with distilled water. The selected transformants were verified for the target gene fragment inserted into the eel locus or ace1 locus by PCR to obtain a target transformant.

A double transformant that expresses mutated XYR1 (V821F) and ACE3 or its variant was produced. An XYR1 (V821F) expression strain into which plasmid 20 had been introduced was cultured using a PDA medium containing 0.2% 5-fluoroorotic acid (5-FOA) monohydrate to select again a strain that grows by acquiring resistance to 5-FOA. The grown strain was acquired as a JN13_XYR1 (V821F) Δpyr4 strain. The acquired strains were transformed again with the plasmids (plasmids 1 to 19) for ACE3 expression. These strains express mutated XYR1 (V821F) and ACE3 or its variant.

### Example 3 Culture of modified filamentous fungus

The filamentous fungus strains obtained in Example 2 were each cultured to produce proteins. In the pre-culture, 50 mL of a medium was placed in a 500-mL flask, the spores of the strains produced in Example 2 were inoculated at 1×10⁵ spores/mL, and shaking culture was performed at 28°C and at 220 rpm (PRXYg-98R, manufactured by Preci Co., Ltd.). The medium composition of the pre-culture is as follows: 1% glucose, 0.14% (NH₄)₂SO₄, 0.2% KH₂PO₄, 0.03% CaCl₂·2H₂O, 0.03% MgSO₄·7H₂O, 0.1% Hipolypepton N, 0.05% Bacto Yeast extract, 0.1% Tween 80, 0.1% Trace element 2, and 50 mM tartaric acid buffer (pH 4.0) (% is w/v% in every ingredient). The composition of the trace element 2 was as follows: 6 mg H₃BO₃, 26 mg (NH₄) ₆Mo₇O₂₄·4H₂O, 100 mg FeCl₃·6H₂O, 40 mg CuSO₄·5H₂O, 8 mg MnCl₂·4H₂O, and 200 mg ZnCl₂ were diluted up to 100 mL with distilled water.

After the pre-culture for 2 days, main culture was performed. 50 mL of a medium was placed in a 500-mL flask, the pre-culture solution was inoculated at 1% (v/v%), and culture was performed at 28°C at 220 rpm for 4 days. The medium composition of main culture was as follows: 3% glucose, 0.14% (NH₄)₂SO₄, 0.2% KH₂PO₄, 0.03% CaCl₂·2H₂O, 0.03% MgSO₄·7H₂O, 0.1% Hipolypepton N, 0.05% Bacto Yeast extract, 0.1% Tween 80, 0.1% Trace element 2, 1.28% diammonium hydrogen citrate, and 50 mM tartaric acid buffer (pH 4.0) (% is w/v% in every ingredient).

### Example 4 Effect of constitutive expression of DBD deletion ACE3 on protein production

The transformants that steadily express *T. reesei-*derived full-length ACE3 or a DBD deletion ACE3 variant produced in Example 2 were evaluated for the protein productivity and the composition of the produced protein.

### 1) Evaluation of protein productivity

The concentrations of the proteins of the cultures in Example 3 were measured by a bradford method. In the bradford method, Quick Start Protein Assay (Bio-Rad Laboratories, Inc.) was used, and each protein amount was calculated based on a calibration curve formed using bovine γ-globulin as a standard protein. The relative protein productivity of each strain when the protein productivity of the JN13 in the culture using glucose only as the carbon source (glucose culture) was defined as 1 was calculated.

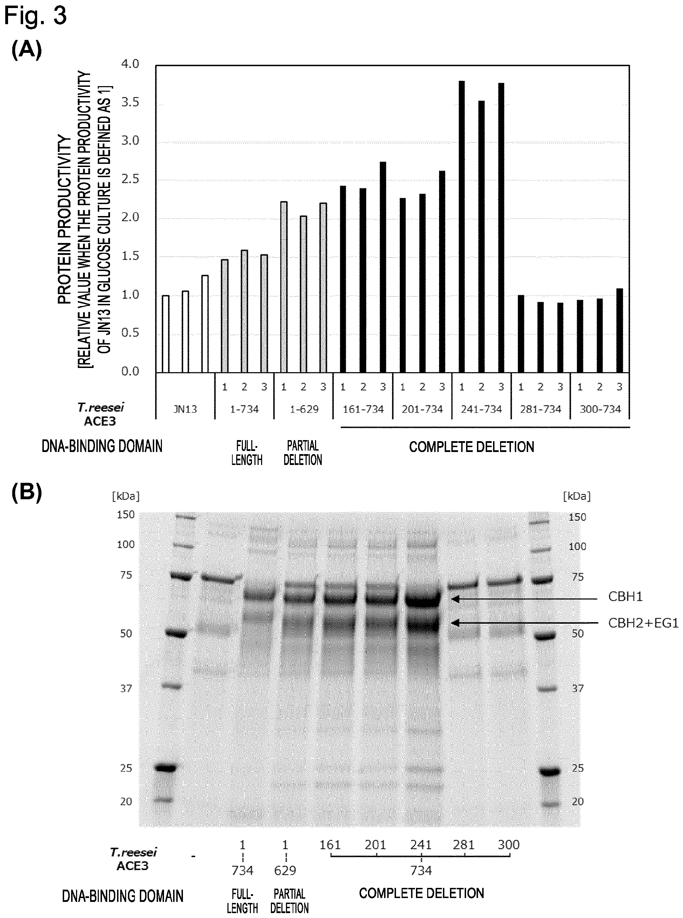

Figure 3(A) shows the relative protein productivity of each transformant. As shown in Figure 3(A), in comparison with a full-length ACE3 (TrACE3 (1-734); SEQ ID NO: 1) expression strain ("1-734") and an ACE3 variant (TrACE3(1-629); SEQ ID NO: 2) expression strain ("1-629") in which a part (C2) of DBD was deleted, the protein productivity was improved in strains ("161-734", "201-734", and "241-734") that express complete DBD deletion ACE3 variants (TrACE3(161-734), TrACE3(201-734), and TrACE3(241-734)) in which 160 to 240 amino acids on the N-terminal side in the sequence of SEQ ID NO: 1 were deleted, the protein productivity was improved. In particular, when 240 amino acids on the N-terminal side of the sequence of SEQ ID NO: 1 were deleted ("241-734"), the protein productivity was significantly improved to exhibit productivity about 1.6 times that of the partial DBD deletion strain ("1-629"). In contrast, in the strains ("281-734" and "300-734") that express ACE3 variants (TrACE3(281-734) and TrACE3(300-734)) in which 280 or more amino acids on the N-terminal side in the sequence of SEQ ID NO: 1 were deleted, the effect of improving protein productivity disappeared.

### 2) Protein composition analysis

The protein composition of each of the cultures in Example 3 was analyzed. The analysis used Mini PROTEAN TGX Stain-Free Gels (Any KD, 15 wells, Bio-Rad Laboratories, Inc.). As the standard, Precision Plus Protein Unstained standards was used. The culture of Example 3 appropriately diluted was mixed with a buffer, treated at 99°C for 5 minutes, and applied to a gel, followed by electrophoresis at 200 V for 35 minutes. The band intensity ratios were calculated from the resulting image file using analysis software (Image Lab), and the composition ratios of the produced saccharification enzymes were calculated.

The results of SDS-PAGE are shown in Figure 3(B). In the DBD deletion ACE3 variant-expressing strains (in Figure 3(B), "161-734", "201-734", and "241-734") having improved protein productivity shown in Figure 3(A), increases in CBH1, CBH2, and EG1 which are main cellulases were observed. That is, it was demonstrated that an improvement in protein productivity by a DBD deletion ACE3 variant is mainly due to an increase in a cellulase component.

### Example 5 Effect of DBD deletion in ACE3 derived from other Trichoderma fungus

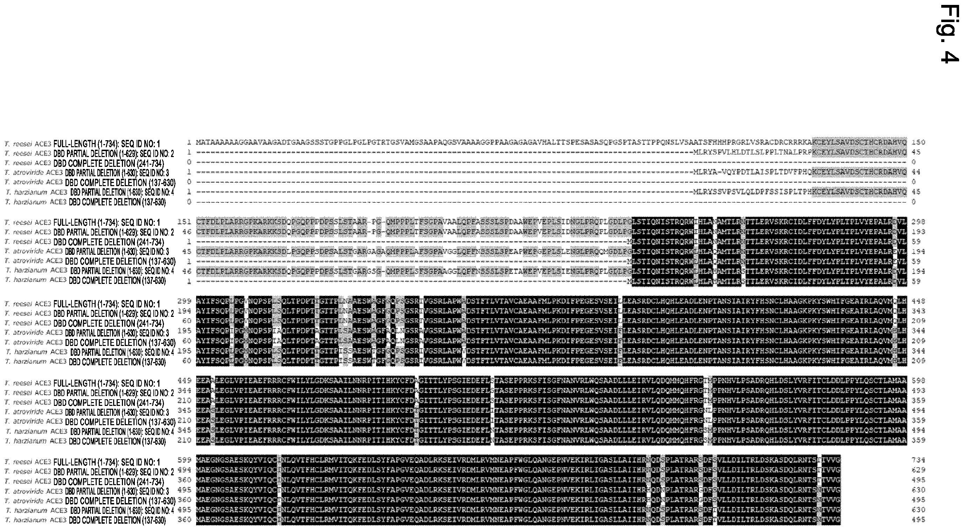

Figure 4 shows the alignment of amino acid sequences of partial DBD deletion ACE3 variants (TrACE3(1-629): SEQ ID NO: 2, TaACE3(1-630): SEQ ID NO: 3, and ThACE3(1-630): SEQ ID NO: 4) which are expressed by the transformants produced in Example 2 and derived from *T. reesei, T. atroviride,* or *T. harzianum* and complete DBD deletion ACE3 variants (TrACE3(241-734), TaACE3(131-630), and ThACE3(131-630)) with respect to *T. reesei* full-length ACE3 (TrACE3(1-734): SEQ ID NO: 1). The sequence alignment demonstrated that the sequences of the partial DBD deletion ACE3 variants (TaACE3(1-630) and ThACE3(1-630)) derived from *T. atroviride* or *T. harzianum* are equivalent to the sequence of the *T*. reesei-derived partial DBD deletion ACE3 variant (TrACE3(1-629)). Similarly, it was demonstrated that the sequences of the complete DBD deletion ACE3 variants (TaACE3(131-630) and ThACE3(131-630)) derived from *T. atroviride* or *T. harzianum* are equivalent to the sequence of the *T.* reesei-derived partial DBD deletion ACE3 variant (TrACE3(241-734)) in which 240 amino acids on the N-terminal side in SEQ ID NO: 1 were deleted.

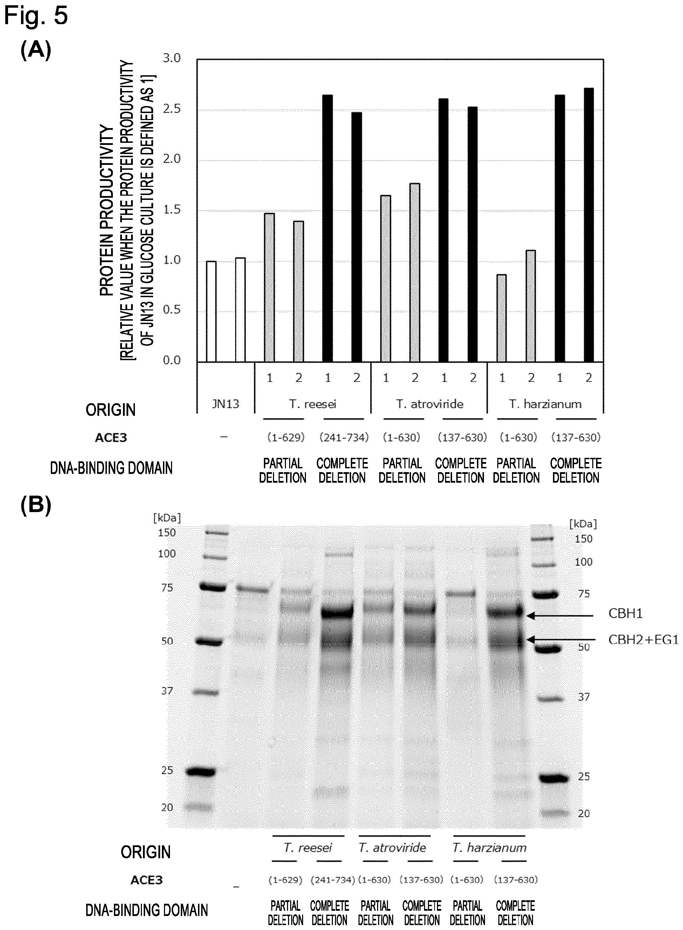

The protein productivity of the transformants that constitutively express the partial or complete DBD deletion ACE3 variants derived from *T. reesei, T. atroviride,* or *T. harzianum* was evaluated by the same method as in Example 4, 1). The results are shown in Figure 5(A). Also in ACE3 of *T. atroviride* and *T. harzianum,* as in ACE3 of *T. reesei,* the protein productivity was improved in a complete DBD deletion ACE3 variant-expressing strain, compared to a partial DBD deletion ACE3 variant-expressing strain. In addition, SDS-PAGE was performed by the same method as in Example 4, 2). As a result, as shown in Figure 5(B), increases in CBH1, CBH2, and EG1 which are main cellulases were observed as in Figure 3(B). Accordingly, it was demonstrated that the improvement of cellulase productivity by a DBD deletion ACE3 variant is caused by not only ACE3 of *T. reesei* but also ACE3 of other *Trichoderma* fungi.

### Example 6 Synergistic effect of DBD deletion and C-terminal deletion ACE3 constitutive expression

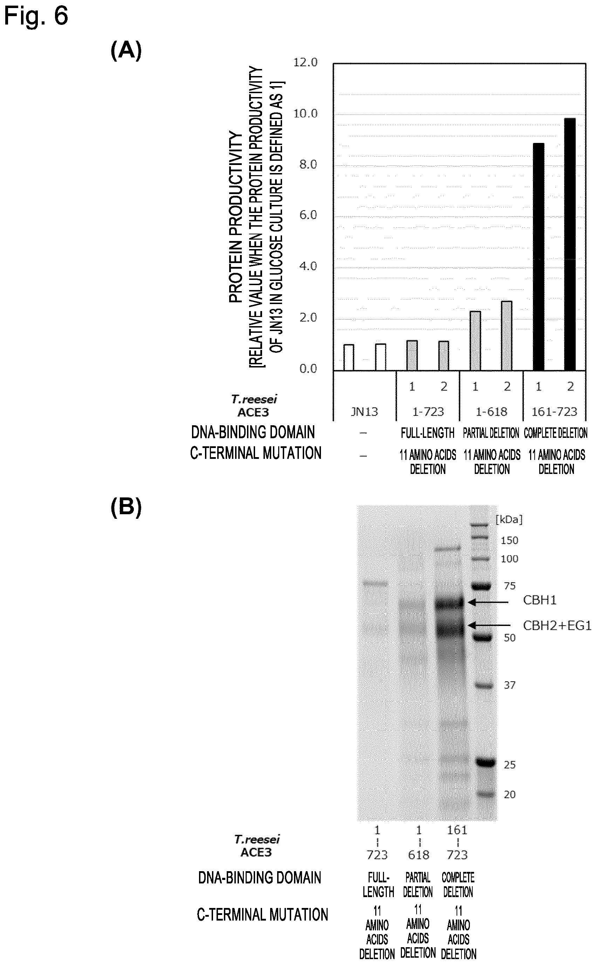

An effect of improving cellulase productivity by C-terminal deletion of ACE3 has been reported (Patent Literature 4 and Non Patent Literature 5). In this Example, regarding the transformants constitutively expressing the ACE3 variant (TrACE3(1-723)) in which 11 amino acids on the C-terminal of the sequence of SEQ ID NO: 1 were deleted, the ACE3 variant (TrACE3(1-618)) having C-terminal deletion and partial DBD deletion, and the ACE3 variant (TrACE3(161-723)) having C-terminal deletion and complete DBD deletion produced in Example 2, the protein productivity was evaluated by the same method as in Example 4, 1). The results are shown in Figure 6(A). Even if there is similar C-terminal deletion, the protein productivity was notably improved in strains expressing the complete DBD deletion ACE3 variant, compared to strains expressing ACE3 variants not having N-terminal deletion or having partial DBD deletion. Furthermore, Figures 3(A) and 6(A) demonstrate that an unexpected high synergistic effect on protein productivity is obtained by combination of DBD deletion and C-terminal deletion. In addition, SDS-PAGE was performed by the same method as in Example 4, 2). As a result, as shown in Figure 6(B), increases in CBH1, CBH2, and EG1 which are main cellulases were observed as in Figure 3(B). Accordingly, it was revealed that complete DBD deletion in ACE3 exerts a higher effect of improving the cellulase productivity than C-terminal deletion and further that the effect is synergistically enhanced by combination of complete DBD deletion and C-terminal deletion.

### Example 7 Effect by combination of DBD deletion ACE3 and mutant XYR1 constitutive expression

The mutated XYR1 (V821F) expression strain and the double transformant constitutively expressing mutated XYR1 (V821F) and ACE3 or its variant produced in Example 2 were evaluated for the protein productivity by the same method as in Example 4, 1) and 2).

The protein productivity was improved in the mutated XYR1 (V821F) expression strain compared to the JN13 strain. In the double transformant, although the improvement in the protein productivity of the full-length ACE3 (TrACE3(1-734)) co-expression strain was slight, compared to the mutated XYR1 (V821F) single expression strain, the protein productivity of the partial DBD deletion ACE3 variant (TrACE3(1-629)) co-expression strain was improved by about 1.5 times. The protein productivity of a co-expression strain of V821F and a complete DBD deletion ACE3 variant (TrACE3(161-734), TrACE3(201-734), or TrACE3(241-734)) was equivalent to that of a co-expression strain of V821F and a partial DBD deletion variant (TrACE3(1-629)), and no further increase in the production quantity was observed.

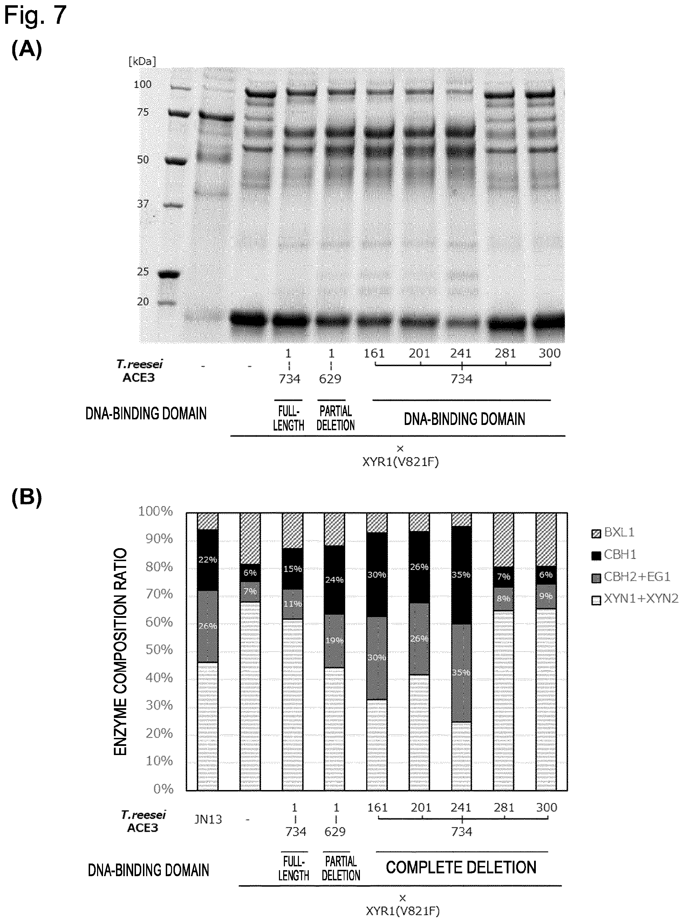

The results of SDS-PAGE are shown in Figure 7(A). The composition ratios of the produced saccharification enzymes are shown in Figure 7(B). In the strain that constitutively expresses mutant XYR1 and a complete DBD deletion ACE3 variant, not only the protein productivity was improved, but also the content ratios of CBH1, CBH2, and EG1 as main cellulases were increased. In contrast, similar to the results of Example 4 (Figure 3), in strains that express ACE3 variants in which 280 or more amino acids on the N-terminal side are deleted, the effects of improving the protein productivity and the cellulase content ratio disappeared. Accordingly, it was demonstrated that the protein productivity and the cellulase content in the produced protein can be further improved by co-expression of mutant XYR1 and a specific complete DBD deletion ACE3 variant, compared to existing co-expression of mutant XYR1 and ACE3 or its variant and therefore that more efficient cellulase production is possible.

### Example 8 Effect of combination of DBD deletion and C-terminal deletion ACE3 and mutant XYR1 constitutive expression

As in the procedure of Example 7, the double transformant constitutively expressing mutated XYR1 (V821F) and a DBD and C-terminal deletion ACE3 variant produced in Example 2 was evaluated for the protein productivity by the same method as in Example 4, 1) and 2).

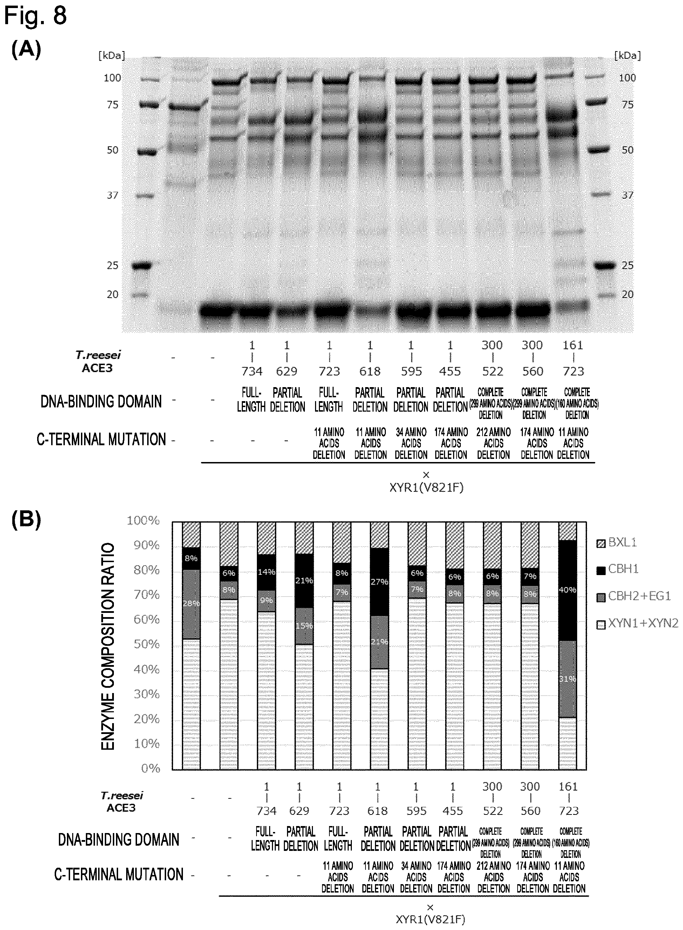

The results of SDS-PAGE are shown in Figure 8(A). The composition ratios of the produced saccharification enzymes are shown in Figure 8(B). The strain that constitutively expresses mutated XYR1 and a complete DBD and C-terminal deletion ACE3 variant (TrACE3(161-723)) exhibited high protein productivity and the highest cellulase content ratio. It was demonstrated that also in combination of mutated XYR1 and C-terminal deletion, complete DBD deletion of ACE3 is effective for improvement in the cellulase content ratio. In contrast, in other C-terminal deletion ACE3 variants (TrACE3(1-723), TrACE3(1-618), TrACE3(1-595), TrACE3(1-455), TrACE3(300-522), and TrACE3(300-560)), no increase in the protein production quantity was observed, compared to a partial DBD deletion ACE3 variant (TrACE3(1-629)) not having C-terminal deletion.

As described above, constitutive expression of an ACE3 variant in which the Zn(II)₂Cys₆-type DNA-binding domain (DBD) had been completely deleted significantly improved the protein productivity of a microorganism in the absence of an inducer. Such an ACE3 variant is effective not only in *T. reesei* but also in other *Trichoderma* fungi and is effective also in combination with an existing effective mutation such as C-terminal deletion or mutated XYR1 (e.g., V821F). Accordingly, it was demonstrated that constitutive expression of the complete DBD deletion ACE3 variant can highly activate a cellulase promoter in a microorganism and achieve highly efficient protein production in the culture using a cellulase noninducible carbon source such as glucose.

## Claims

1. A modified filamentous fungus expressing an ACE3 variant, wherein
the ACE3 variant is a variant in which substantially the whole of a Zn(II)₂Cys₆-type DNA-binding domain of ACE3 is deleted.

2. The modified filamentous fungus according to claim 1, wherein
the ACE3 is a polypeptide consisting of an amino acid sequence of any one of SEQ ID NOs: 1 to 4 or an amino acid sequence having at least 90% identity thereto.

3. The modified filamentous fungus according to claim 2, wherein
the Zn(II)₂Cys₆-type DNA-binding domain is a region corresponding to amino acids at positions 120 to 160 in SEQ ID NO: 1.

4. The modified filamentous fungus according to claim 2, wherein
the ACE3 variant has deletion of a region corresponding to amino acids at positions 1 to 160 in SEQ ID NO: 1.

5. The modified filamentous fungus according to any one of claims 2 to 4, wherein
the ACE3 variant includes a region corresponding to amino acids at positions 280 to 701 in SEQ ID NO: 1.

6. The modified filamentous fungus according to any one of claims 2 to 5, wherein
the ACE3 variant has deletion of a region corresponding to 11 amino acids on the C-terminal in the amino acid sequence of SEQ ID NO: 1.

7. The modified filamentous fungus according to any one of claims 1 to 6, wherein a gene that expresses the ACE3 variant has been introduced into the modified filamentous fungus.

8. The modified filamentous fungus according to claim 7, wherein
the gene that expresses the ACE3 variant is operably linked to a control region that promotes transcription of the gene.

9. The modified filamentous fungus according to any one of claims 1 to 8, wherein
the filamentous fungus is a *Trichoderma* fungus.

10. A method for producing a protein, comprising culturing the modified filamentous fungus according to any one of claims 1 to 9.

11. The method according to claim 10, wherein the protein is cellulase and/or hemicellulase.

12. The method according to claim 10 or 11, wherein the culture is performed in the presence of glucose.

13. A method for producing a modified filamentous fungus, comprising:
modifying a parent filamentous fungus so as to express an ACE3 variant, wherein
the ACE3 variant is a variant in which substantially the whole of a Zn(II)₂Cys₆-type DNA-binding domain of ACE3 is deleted.

14. The method according to claim 13, wherein
the ACE3 is a polypeptide consisting of the amino acid sequence of SEQ ID NO: 1 or an amino acid sequence having at least 90% identity thereto.

15. The method according to claim 14, wherein
the Zn(II)₂Cys₆-type DNA-binding domain is a region corresponding to amino acids at positions 120 to 160 in SEQ ID NO: 1.

16. The method according to claim 14, wherein
the ACE3 variant has deletion of a region corresponding to amino acids at positions 1 to 160 in SEQ ID NO: 1.

17. The method according to any one of claims 14 to 16, wherein
the ACE3 variant includes a region corresponding to amino acids at positions 280 to 701 in SEQ ID NO: 1.

18. The method according to any one of claims 14 to 17, wherein
the ACE3 variant has deletion of a region corresponding to 11 amino acids on the C-terminal in the amino acid sequence of SEQ ID NO: 1.

19. The method according to any one of claims 13 to 18, wherein
modification of the parent filamentous fungus includes introduction of a gene that expresses the ACE3 variant into the parent filamentous fungus.

20. The method according to claim 19, wherein
the gene that expresses the ACE3 variant is operably linked to a control region that promotes transcription of the gene.

21. The method according to any one of claims 13 to 20, wherein
the filamentous fungus is a *Trichoderma* fungus.
